# EUROPEAN PATENT APPLICATION

(11) **EP 4 023 068 A1**
(43) Date of publication of application: **06.07.2022**
(21) Application number: 20872255.3
(22) Date of filing: 30.09.2020
(51) Int. Cl.: A01N 59/08, A01P 3/00, A61K 9/20, A61K 9/28, A61K 9/48, A61K 9/54, A61K 31/122, A61K 33/00, A61K 33/40, A61K 47/02, A61P 1/00, A61P 1/02, A61P 1/04, A61P 31/04, A61P 31/12

(54) **DRUG, DRUG MANUFACTURING METHOD, AND WATER PURIFICATION METHOD**

(30) Priority: 01.10.2019 JP 2019181759
(71) Applicant: Acenet Inc., Tokyo 105-0021 (JP)
(72) Inventor: TAKAMORI Kiyoto, Tokyo 105-0021 (JP); SHIBATA Takekatsu, Tokyo 105-0021 (JP); KONISHI Kiyoshi, Tokyo 105-0021 (JP)
(74) Representative: J A Kemp LLP
(86) International application number: PCT/JP2020/037370
(87) International publication number: WO 2021/066080

(57) **Abstract**

It is an object of the present invention to provide a solid drug that is very convenient to transport and store. In order to achieve the object, a drug according to the present invention is a solid drug that includes a radical generating catalyst and a radical generation source.

## Description

### TECHNICAL FIELD

The present invention relates to a drug, a drug manufacturing method, and a water purification method.

### BACKGROUND ART

Radicals are widely-used important chemical species because they have high reactivity. For example, sodium chlorite (NaClO₂) is a non-toxic and inexpensive oxidizing agent, and has been used as a precursor of a chlorine dioxide radical (ClO₂) (Non-Patent Literature 1 to 4).

Liquid drugs that utilize the reactivity of radicals and are highly safe and highly disinfectant have been proposed (Patent Literature 1 and 2).

### Citation List

### Non-Patent Literature

Non-Patent Literature 1: H. Dodgen and H. Taube, J. Am. Chem. Soc., 1949, 71, 2501-2504.
Non-Patent Literature 2: J. K. Leigh, J. Rajput, and D. E. Richardson, Inorg. Chem., 2014, 53,6715-6727.
Non-Patent Literature 3: C. L. Latshaw, Tappi, 1994, 163-166.
Non-Patent Literature 4: (a) J. J. Leddy, in Riegel's Handbook of Industrial Chemistry, 8th edn. Ed., J. A. Kent, Van Nostrand Reinhold Co. Inc, New York, 1983, pp. 212-235; (b) I. Fabian, Coord. Chem. Rev., 2001, 216-217, 449-472.
Patent Literature 1: Japanese Patent No. 6236057
Patent Literature 2: WO 2018/230743A1

### SUMMARY OF INVENTION

### Technical Problem

Liquid drugs can be used for spraying (atomizing), application, and the like, and are thus very convenient to use. However, solid drugs are more convenient to transport and store.

Therefore, it is an object of the present invention to provide a solid drug that is very convenient to transport and store, a method for manufacturing the drug, and a method for purifying water using the drug.

### Solution to Problem

In order to achieve the object mentioned above, a drug according to the present invention is a drug in a solid form that includes: a radical generating catalyst; and a radical generation source.

A drug manufacturing method according to the present invention is a method for manufacturing the drug according to the present invention that includes a pressing step of pressing a composition containing constituent components of the drug according to the present invention into a solid form.

A water purification method according to the present invention is a water purification method that includes a step of adding the drug according to the present invention, or a drug-containing solution containing the drug according to the present invention, into water. Advantageous Effects of Invention

With the present invention, it is possible to provide a solid drug that is very convenient to transport and store, a method for manufacturing the drug, and a method for purifying water using the drug.

### DESCRIPTION OF EMBODIMENTS

Hereinafter, the present invention will be described more specifically by way of embodiments. However, the present invention is not limited to the description below.

### 1. Drug and Components Thereof

As described above, a drug according to the present invention is a solid drug characterized by including a radical generating catalyst and a radical generation source. The drug according to the present invention may or may not include another component in addition to the radical generating catalyst and the radical generation source. The drug according to the present invention may include, for example, a stabilizer and the like, which will be described later.

Note that the drug according to the present invention may be a solid drug characterized by including, for example, at least one of a buffer and a liquid property-controlling agent, and a radical generation source, unlike the case above. In this case, the drug above may or may not include a radical generating catalyst. Note that specific examples of the radical generation source, the radical generating catalyst, the buffer, and the liquid property-controlling agent will be described later.

There is no particular limitation on the use of the drug according to the present invention. For example, the drug may be dissolved or dispersed in a medium (e.g., water) and then used in the form of a liquid drug, as described later. It is more convenient to transport and store a solid drug and dissolve or disperse the solid drug in a medium to produce a liquid drug before use rather than transport and store a liquid drug as it is. The solid drug according to the present invention may be, for example, in the form of a tablet. Also, for example, a solid drug (e.g., tablet) made of a radical generation source and a solid drug (e.g., tablet) made of a radical generating catalyst may be individually formed, dissolved or dispersed in the same medium (e.g., water) before use, and used in the form of a liquid drug. Degradation of a radical generation source by a radical generating catalyst in a solid drug can be prevented by individually solidifying the radical generation source and the radical generating catalyst. On the other hand, the radical generating catalyst can act to generate a radical from the radical generation source by dissolving or dispersing the radical generating catalyst and the radical generation source in the same medium before use. Also, for example, a radical generation source and a radical generating catalyst may be respectively formed into two layers (multiple layers) that are separated from each other in a single solid drug through coating or micro-encapsulation, which will be described later.

### 1-1. Radical Generating Catalyst

A radical generating catalyst used in the drug according to the present invention (referred to as a "radical generating catalyst according to the present invention" hereinafter) may be, for example, an organic compound or an inorganic substance.

The radical generating catalyst according to the present invention may include, for example, an ammonium salt. The ammonium salt may be an organic ammonium salt or an inorganic ammonium salt, or may include both. The inorganic ammonium salt is not particularly limited, and may be a NH₄⁺ salt (e.g., NH₄Cl).

The radical generating catalyst according to the present invention may include at least one selected from the group consisting of an amino acid, a protein, a peptide, a phospholipid, and salts thereof. Specific examples of the amino acid, the protein, the peptide, and the phospholipid will be described later. In this case, the radical generating catalyst may further include, for example, the above-mentioned ammonium salt.

In the radical generating catalyst according to the present invention, the inorganic substance may include one or both of a metal ion and a non-metal ion. The metal ion may include one or both of a typical metal ion and a transition metal ion. The inorganic substance may be, for example, at least one selected from the group consisting of an alkali earth metal ion, a rare earth ion, Sc³⁺, Li⁺, Fe²⁺, Fe³⁺, Al³⁺, a silicate ion, and a borate ion. Examples of the alkali earth metal ion include a calcium ion, a strontium ion, a barium ion, and a radium ion, and more specific examples thereof include Ca²⁺, Sr²⁺, Ba²⁺, and Ra²⁺. "Rare earth" is a general term for seventeen elements in total, namely two elements including scandium ₂₁Sc and yttrium ₃₉Y and fifteen elements (lanthanoids) from lanthanum ₅₇La to lutetium ₇₁Lu. Examples of the rare earth ion include trivalent positive ions of the respective seventeen elements above.

The Lewis acid (including a counter ion) may be, for example, at least one selected from the group consisting of CaCl₂, MgCh, FeCh, FeCl₃, AlCl₃, AlMeCl₂, AlMe₂Cl, BF₃, BPh₃, BMe₃, TiCl₄, SiF₄, and SiCl₄. Note that "Ph" represents a phenyl group, and "Me" represents a methyl group.

Note that the radical generating catalyst according to the present invention can be selected from the above-listed radical generating catalysts as appropriate depending on the intended use in consideration of the reactivity level, the acidity level, the safety, and the like.

As a result of the study conducted by the inventors of the present invention, they found that an ammonium (particularly an organic ammonium), an amino acid, a protein, a peptide, and a phospholipid function as a radical generating catalyst. Also, as a result of further study, the inventors of the present invention found that an ammonium, an amino acid, a protein, a peptide, and a phospholipid that function as a radical generating catalyst may have Lewis acid-like properties. That is, although the reason why an ammonium, a protein, an amino acid, a peptide, and a phospholipid function as a radical generating catalyst has not been revealed, it is presumed that the reason for this is that the ammonium, amino acid, peptide, and phospholipid have Lewis acid-like functions. Also, as a result of further study, the inventors of the present invention found a radical generating catalyst that includes an organic compound having at least one of the Lewis acid property and the Brønsted acid property. Note that the "Lewis acid" in the present invention is a substance that acts as a Lewis acid to the radical generation source.

The Lewis acidity of the radical generating catalyst according to the present invention is, for example, 0.4 eV or more, 0.5 eV or more, or 0.6 eV or more. The maximum value of the Lewis acidity is not particularly limited, and is, for example, 20 eV or less. In the present invention, if a value obtained through a measurement using either of the "Lewis Acidity Measurement Method (1)" or the "Lewis Acidity Measurement Method (2)", which will be described later, is greater than or equal to, or smaller than or equal to, the above-mentioned values, it may be determined that the Lewis acidity is greater than or equal to, or smaller than or equal to, the above-mentioned values.

The Lewis acidity can be measured using, for example, the method described in Ohkubo, K.; Fukuzumi, S. Chem. Eur. J., 2000, 6, 4532, J. AM. CHEM. SOC. 2002, 124, 10270-10271, or J. Org. Chem. 2003, 68, 4720-4726. Specifically, the Lewis acidity can be measured using the "Lewis Acidity Measurement Method (1)" below.

### Lewis Acidity Measurement Method (1)

Acetonitrile (MeCN) containing cobalt tetraphenylporphyrin shown in Chemical Reaction Formula (1a) below, saturated O₂, and a Lewis acidity measurement target (e.g., a cation of a metal or the like; represented by Mⁿ⁺ in the Chemical Reaction Formula (1a) below) is left to stand at room temperature, and a change in an ultraviolet-visible absorption spectrum is measured. A ΔE value (eV), which is an index for the Lewis acidity, can be calculated from the obtained reaction rate constant (k_{cat}). The larger the k_{cat} value is, the stronger the Lewis acidity is. Also, the Lewis acidity of an organic compound can be estimated from the energy level of the lowest unoccupied molecular orbital (LUMO) that is calculated through quantum chemical calculation. The larger of a positive value the obtained value is, the stronger the Lewis acidity is.

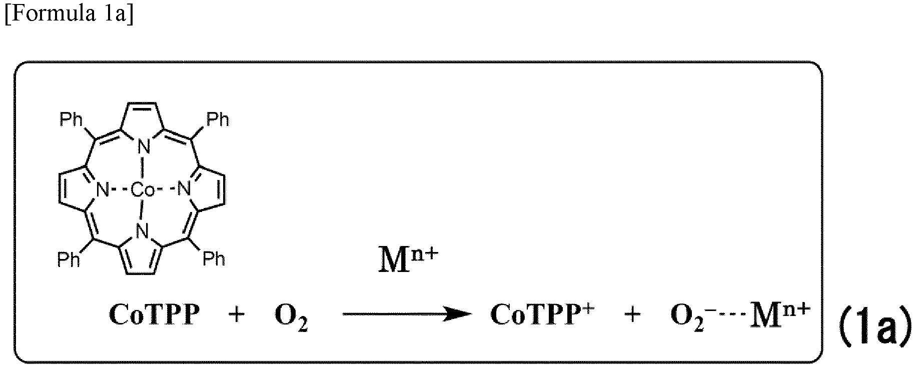

Note that examples of the rate constant for a reaction between CoTPP and oxygen in the presence of a Lewis acid, which is measured (calculated) using the above-mentioned measurement method and can be used as an index for the Lewis acidity, are shown below. In the table below, the "k_{cat}, M⁻²s⁻¹" values are CoTPP and oxygen in the presence of a Lewis acid. The "LUMO, eV" values are the LUMO energy levels. The table below shows the values for "benzetonium chloride", "benzalkonium chloride", "tetramethylammonium hexafluorophosphate", "tetrabutylammonium hexafluorophosphate", and "ammonium hexafluorophosphate".

### [Table tpp]

| | LUMO, eV | *k*_{cat}, M⁻² s⁻¹ |
|---|---|---|
| benzetonium chloride | -4.12 | 0.24 |
| benzalkonium chloride | -4.02 | 0.18 |
| tetramethylammonium hexafluorophosphate | -3.58 | > 0.1 |
| tetrabutylammonium hexafluorophosphate | -2.07 | > 0.1 |
| ammonium hexafluorophosphate | -5.73 | 20 |

Also, in the present invention, the Lewis acidity may be measured in the same manner as the Lewis acidity measurement method (1), except that ubiquinone 1 (Q1) is used instead of an oxygen molecule (O₂), and the ubiquinone 1 is reduced to produce a ubiquinone 1 anion radical. Such a Lewis acidity measurement method may also be referred to as a "Lewis acidity measurement method (2)" hereinafter. In the Lewis acidity measurement method (2), the measurement can be performed in the same manner as the Lewis acidity measurement method (1), except that ubiquinone 1 (Q1) is used instead of an oxygen molecule (O₂). Also, in the Lewis acidity measurement method (2), a ΔE value (eV), which is an index for the Lewis acidity, can be calculated from the obtained reaction rate constant (k_{cat}) as in the Lewis acidity measurement method (1). The Lewis acidity measurement method (2) is described in, for example, Ohkubo, K.; Fukuzumi, S. Chem. Eur. J., 2000, 6, 4532, and can be performed in accordance with, or in conformity with, the method described in this literature.

The Lewis acidity measurement method (2) can be performed by measuring the reaction rate constant (k_{cat}) for Chemical Reaction Formula (1b) below.

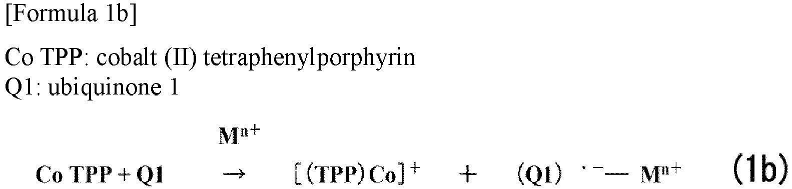

In the Chemical Reaction Formula (1b) above,
Mⁿ⁺ represents the above-mentioned radical generating catalyst,
CoTPP represents cobalt (II) tetraphenylporphyrin,
Q1 represents ubiquinone 1,
[(TPP)Co]⁺ represents a cobalt (III) tetraphenylporphyrin cation, and
(Q1)^{·-} represents a ubiquinone 1 anion radical.

The Lewis acidity of the radical generating catalyst according to the present invention is determined as, for example, the reaction rate constant (k_{cat}) for the Chemical Reaction Formula (1b) above, that is, the measurement value (K_{obs}) of the reaction rate constant (k_{cat}) measured using the "Lewis acidity measurement method (2)", and may be, for example, 1.0×10⁻⁵ S⁻¹ or more, 2.0×10⁻⁵ S⁻¹ or more, 3.0×10⁻⁵ S⁻¹ or more, 4.0×10⁻⁵ S⁻¹ or more, 5.0×10⁻⁵ S⁻¹ or more, 6.0×10⁻⁵ S⁻¹ or more, 7.0×10⁻⁵ S⁻¹ or more, 8.0×10⁻⁵ S⁻¹ or more, 9.0×10⁻⁵ S⁻¹ or more, 1.0×10⁻⁴ S⁻¹ or more, 2.0×10⁻⁴ S⁻¹ or more, 3.0×10⁻⁴ S⁻¹ or more, 4.0×10⁻⁴ S⁻¹ or more, 5.0×10⁻⁴ S⁻¹ or more, 6.0×10⁻⁴ S⁻¹ or more, 7.0×10⁻⁴ S⁻¹ or more, 8.0×10⁻⁴ S⁻¹ or more, 9.0×10⁻⁴ S⁻¹ or more, 1.0×10⁻³ S⁻¹ or more, 2.0×10⁻³ S⁻¹ or more, 3.0×10⁻³ S⁻¹ or more, 4.0×10⁻³ S⁻¹ or more, 5.0×10⁻³ S⁻¹ or more, 6.0×10⁻³ S⁻¹ or more, 7.0×10⁻³ S⁻¹ or more, 8.0×10⁻³ S⁻¹ or more, 9.0×10⁻³ S⁻¹ or more, 1.0×10⁻² S⁻¹ or more, 2.0×10⁻² S⁻¹ or more, 3.0×10⁻² S⁻¹ or more, 4.0×10⁻² S⁻¹ or more, 5.0×10⁻² S⁻¹ or more, 6.0×10⁻² S⁻¹ or more, 7.0×10⁻² S⁻¹ or more, 8.0×10⁻² S⁻¹ or more, or 9.0×10⁻² S⁻¹ or more, and 1.0×10⁻¹ S⁻¹ or less, 9.0×10⁻² S⁻¹ or less, 8.0×10⁻² S⁻¹ or less, 7.0×10⁻² S⁻¹ or less, 6.0×10⁻² S⁻¹ or less, 5.0×10⁻² S⁻¹ or less, 4.0×10⁻² S⁻¹ or less, 3.0×10⁻² S⁻¹ or less, 2.0×10⁻² S⁻¹ or less, 1.0×10⁻² S⁻¹ or less, 9.0×10⁻³ S⁻¹ or less, 8.0×10⁻³ S⁻¹ or less, 7.0×10⁻³ S⁻¹ or less, 6.0×10⁻³ S⁻¹ or less, 5.0×10⁻³ S⁻¹ or less, 4.0×10⁻³ S⁻¹ or less, 3.0×10⁻³ S⁻¹ or less, 2.0×10⁻³ S⁻¹ or less, 1.0×10⁻³ S⁻¹ or less, 9.0×10⁻⁴ S⁻¹ or less, 8.0×10⁻⁴ S⁻¹ or less, 7.0×10⁻⁴ S⁻¹ or less, 6.0×10⁻⁴ S⁻¹ or less, 5.0×10⁻⁴ S⁻¹ or less, 4.0×10⁻⁴ S⁻¹ or less, 3.0×10⁻⁴ S⁻¹ or less, 2.0×10⁻⁴ S⁻¹ or less, 1.0×10⁻⁴ S⁻¹ or less, 9.0×10⁻⁵ S⁻¹ or less, 8.0×10⁻⁵ S⁻¹ or less, or 7.0×10⁻⁵ S⁻¹ or less.

In the radical generating catalyst according to the present invention, the ammonium may be, for example, a quaternary ammonium, tertiary ammonium, secondary ammonium, primary ammonium, or unsubstituted ammonium. The ammonium is not particularly limited, and may be, for example, a nucleic acid base or the like, or an amino acid or peptide, which will be described later.

In the radical generating catalyst according to the present invention, at least one selected from the group consisting of an ammonium, an amino acid, a peptide, a phospholipid, and salts thereof (a first radical generating catalyst according to the present invention) or a compound having at least one of the Lewis acid property and the Brønsted acid property (a second radical generating catalyst according to the present invention) may be, for example, a cationic surfactant including a quaternary ammonium-type cationic surfactant. Examples of the quaternary ammonium-type cationic surfactant include benzalkonium chloride, benzethonium chloride, cetylpyridinium chloride, hexadecyltrimethylammonium bromide, dequalinium chloride, edrophonium, didecyldimethylammonium chloride, tetramethylammonium chloride, tetrabutylammonium chloride, benzyltriethylammonium chloride, oxitropium, carbachol, glycopyrronium, safranine, sinapine, tetraethylammonium bromide, hexadecyltrimethylammonium bromide, suxamethonium, sphingomyelin, ganglioside GM1, denatonium, trigonelline, neostigmine, paraquat, pyridostigmine, phellodendrine, pralidoxime methyl iodide, betaine, betanin, bethanechol, betalain, lecithin, adenine, guanine, cytosine, thymine, uracil, and cholines (e.g., choline chlorides such as benzoylcholine chloride and lauroylcholine chloride hydrate, phosphocholine, acetylcholine, choline, dipalmitoylphosphatidylcholine, and choline bitartrate). However, in the radical manufacturing method according to the present invention, the quaternary ammonium is not limited to a surfactant.

In the radical generating catalyst according to the present invention, the ammonium salt may be, for example, an ammonium salt represented by Chemical Formula (XI) below.

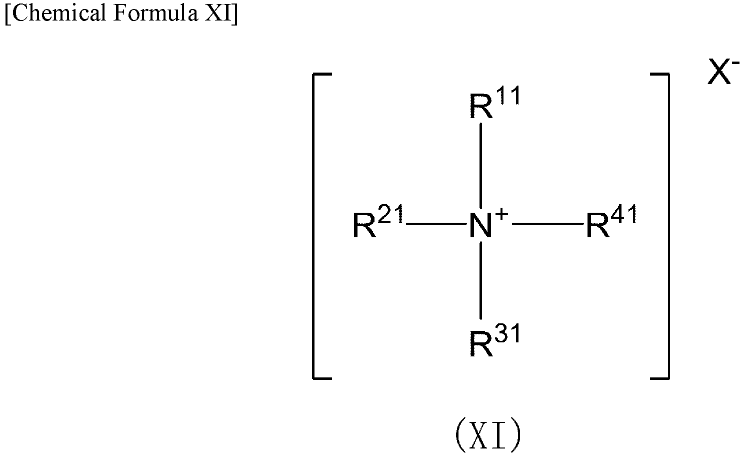

In the Chemical Formula (XI) above,
R¹¹, R²¹, R³¹, and R⁴¹ are independently a hydrogen atom or an aromatic ring, or an alkyl group that optionally includes an ether bond, a carbonyl group, an ester bond, or an amide bond, or an aromatic ring, and are optionally the same or different, or
two or more of R¹¹, R²¹, R³¹, and R⁴¹ are optionally linked to each other to form a ring structure together with N⁺ to which these groups link, the ring structure being optionally saturated or unsaturated, being optionally an aromatic ring or non-aromatic ring, and optionally having one or more substituents, and
X⁻ is an anion. X⁻ is, for example, an anion other than a peroxodisulfate ion.

In R¹¹, R²¹, R³¹, and R⁴¹, the aromatic ring is not particularly limited, and optionally includes a heteroatom, and optionally includes a substituent, for example. Examples of the aromatic ring (heteroaromatic ring) that includes a heteroatom include nitrogen-containing aromatic rings, sulfur-containing aromatic rings, and oxygen-containing aromatic rings. Examples of the aromatic ring that includes no heteroatoms include a benzene ring, a naphthalene ring, an anthracene ring, and a phenanthrene ring. Examples of the heteroaromatic ring include a pyridine ring, a thiophene ring, and a pyrene ring. The nitrogen-containing aromatic ring optionally has, for example, a positive charge. Examples of the nitrogen-containing aromatic ring having no positive charge include a pyrroline ring, a pyridine ring, a pyridazine ring, a pyrimidine ring, a pyrazine ring, a quinoline ring, an isoquinoline ring, an acridine ring, a 3,4-benzoquinoline ring, a 5,6-benzoquinoline ring, a 6,7-benzoquinoline ring, a 7,8-benzoquinoline ring, a 3,4-benzoisoquinoline ring, a 5,6-benzoisoquinoline ring, a 6,7-benzoisoquinoline ring, and a 7,8-benzoisoquinoline ring. Examples of the nitrogen-containing aromatic ring having a positive charge include a pyrrolinium ring, a pyridinium ring, a pyridazinium ring, a pyrimidinium ring, a pyrazinium ring, a quinolinium ring, an isoquinolinium ring, an acridinium ring, a 3,4-benzoquinolinium ring, a 5,6-benzoquinolinium ring, a 6,7-benzoquinolinium ring, a 7,8-benzoquinolinium ring, a 3,4-benzoisoquinolinium ring, a 5,6-benzoisoquinolinium ring, a 6,7-benzoisoquinolinium ring, and a 7,8-benzoisoquinolinium ring. Examples of the oxygen-containing aromatic ring and the sulfur-containing aromatic ring include aromatic rings obtained by substituting at least one of carbon atoms and nitrogen atoms in the aromatic rings that include no heteroatoms and the nitrogen-containing aromatic rings with at least one of an oxygen atom and a sulfur atom.

In R¹¹, R²¹, R³¹, and R⁴¹, when the alkyl group or the aromatic ring has a substituent, the substituent is not particularly limited and is optional, and examples thereof include a sulfo group, a nitro group, and a diazo group.

The ammonium salt represented by Chemical Formula (XI) above may be, for example, an ammonium salt represented by Chemical Formula (XII) below.

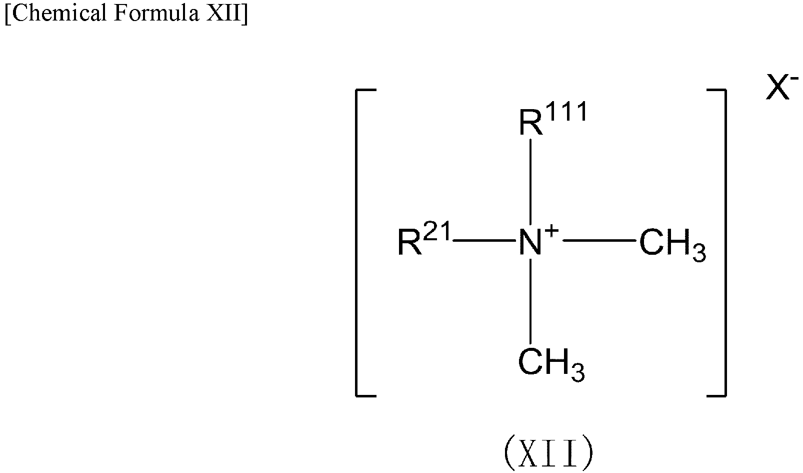

In the Chemical Formula (XII) above,
R¹¹¹ is an alkyl group having 5 to 40 carbon atoms and optionally includes an ether bond, a ketone (carbonyl group), an ester bond, or an amide bond, a substituent, or an aromatic ring, and
R²¹ and X⁻ are the same as those in Chemical Formula (XI) above.

In R¹¹¹, the aromatic ring is not particularly limited, and optionally includes a heteroatom, and optionally includes a substituent, for example. In R¹¹¹, specific examples of the aromatic ring include, but are not particularly limited to, the same aromatic rings as those listed for R¹¹, R²¹, R³¹, and R⁴¹ in Chemical Formula (XI) above.

In R¹¹¹, when the alkyl group or the aromatic ring has a substituent, the substituent is not particularly limited and is optional, and examples thereof include the same substituents as those listed for R¹¹, R²¹, R³¹, and R⁴¹ in Chemical Formula (XI) above.

In the Chemical Formula (XII) above,
R²¹ is optionally, for example, a methyl group or a benzyl group, one or more hydrogen atoms of the benzene ring in the benzyl group is optionally substituted with any substituent, and the substituent is optionally, for example, an alkyl group, an unsaturated aliphatic hydrocarbon group, an aryl group, a heteroaryl group, a halogen, a hydroxy group (-OH), a mercapto group (-SH), or an alkylthio group (-SR, where R is an alkyl group).

The ammonium salt represented by Chemical Formula (XII) above may be, for example, an ammonium salt represented by Chemical Formula (XIII) below.

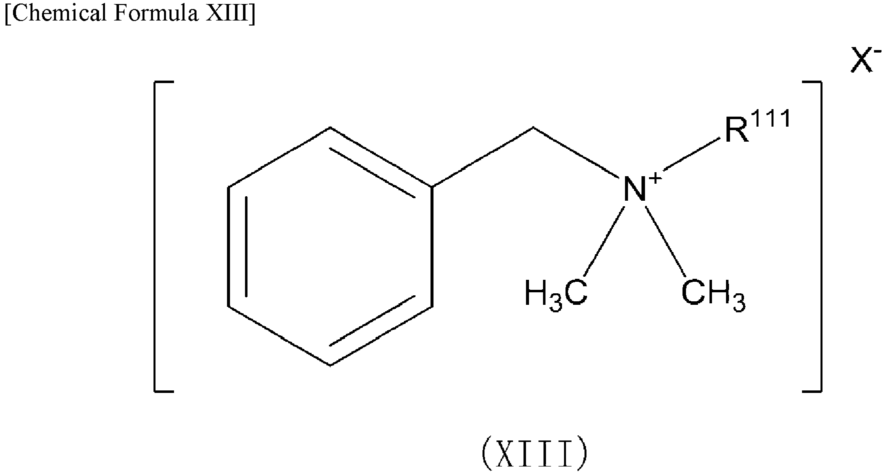

In the Chemical Formula (XIII) above,
R¹¹¹ and X⁻ are the same as those in Chemical Formula (XII) above.

The ammonium represented by Chemical Formula (XI) above may be, for example, an ammonium salt represented by Chemical Formula (XIV) below.

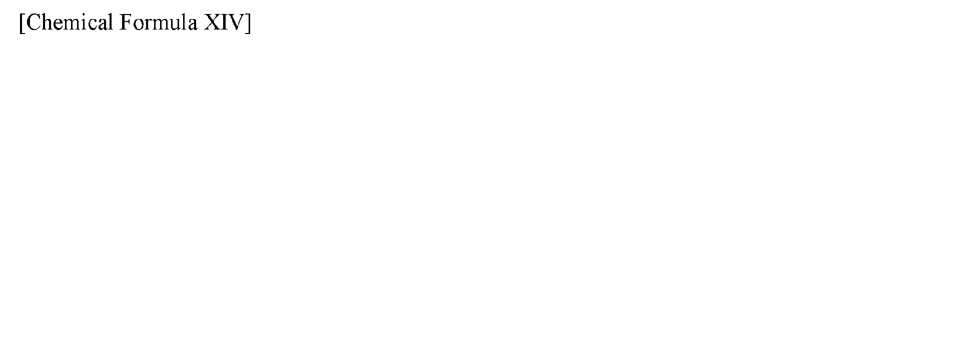

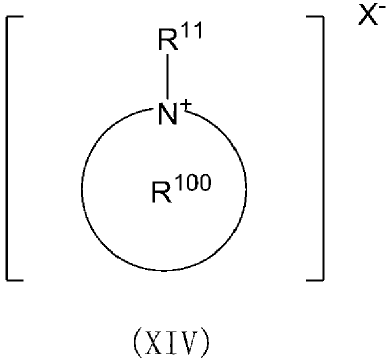

In the Chemical Formula (XIV) above,
R¹⁰⁰ optionally forms a ring structure, the ring structure being optionally saturated or unsaturated, being optionally an aromatic ring or non-aromatic ring, and optionally having one or more substituents, and
R¹¹ and X⁻ are the same as those in Chemical Formula (XI) above.

The ammonium salt represented by Chemical Formula (XI) above may be, for example, an ammonium salt represented by Chemical Formula (XV) below.

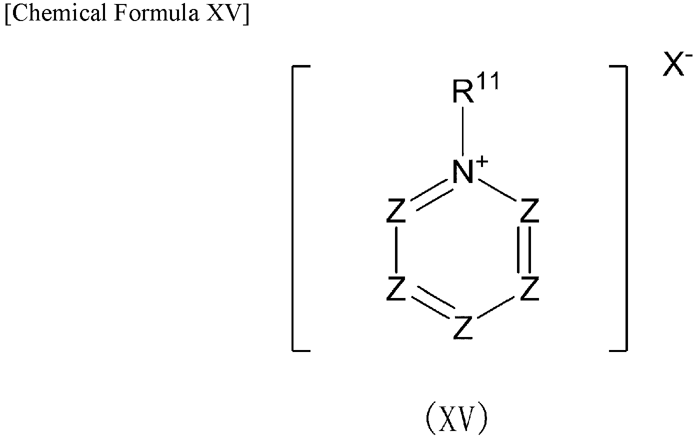

In the Chemical Formula (XV) above,
groups Z are independently CH or N, and are optionally the same or different, H in CH being optionally substituted with a substituent, and
R¹¹ and X⁻ are the same as those in Chemical Formula (XI) above.

The ammonium salt represented by Chemical Formula (XI) above may be, for example, an ammonium salt represented by Chemical Formula (XVI) below.

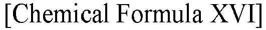

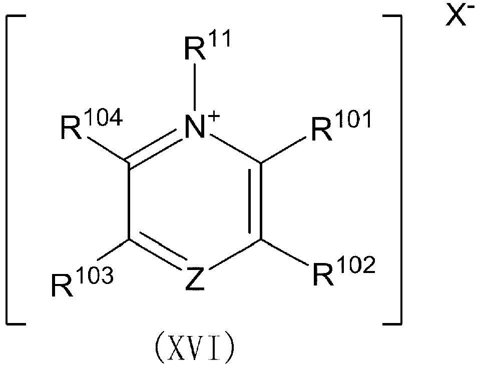

In the Chemical Formula (XVI) above,
R¹⁰¹, R¹⁰², R¹⁰³, and R¹⁰⁴ are independently a hydrogen atom or a substituent, and are optionally the same or different, or
two or more of R¹⁰¹, R¹⁰², R¹⁰³, and R¹⁰⁴ are optionally linked to each other to form a ring structure together with N⁺ to which these groups are linked, the ring structure being optionally saturated or unsaturated, being optionally an aromatic ring or non-aromatic ring, and optionally having one or more substituents,
Z is CH or N, H in CH being optionally substituted with a substituent, and
R¹¹ and X⁻ are the same as those in Chemical Formula (XI) above.

The ammonium salt represented by Chemical Formula (XI) above may be, for example, an ammonium salt represented by Chemical Formula (XVII) below.

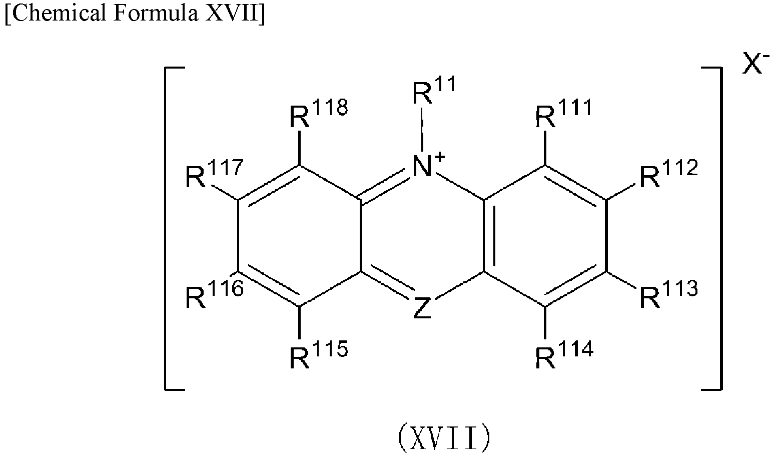

In the Chemical Formula (XVII) above,
R¹¹¹ to R¹¹⁸ are independently a hydrogen atom or a substituent, and are optionally the same or different, or
two or more of R¹¹¹ to R¹¹⁸ are optionally linked to each other to form a ring structure, the ring structure being optionally an aromatic ring or non-aromatic ring, and optionally having one or more substituents,
Z is CH or N, H in CH being optionally substituted with a substituent, and
R¹¹ and X⁻ are the same as those in Chemical Formula (XI) above.

The ammonium salt represented by Chemical Formula (XI) above may be, for example, at least one selected from the group consisting of benzethonium chloride, benzalkonium chloride, hexadecyltrimethylammonium chloride, tetramethylammonium chloride, ammonium chloride, methylammonium chloride, tetrabutylammonium chloride, cetylpyridinium chloride, hexadecyltrimethylammonium bromide, dequalinium chloride, edrophonium, didecyldimethylammonium chloride, benzyltriethylammonium chloride, oxitropium, carbachol, glycopyrronium, safranine, sinapine, tetraethylammonium bromide, hexadecyltrimethylammonium bromide, suxamethonium, sphingomyelin, ganglioside GM1, denatonium, trigonelline, neostigmine, paraquat, pyridostigmine, phellodendrine, pralidoxime methyl iodide, betaine, betanin, bethanechol, betalain, lecithin, adenine, guanine, cytosine, thymine, uracil, and cholines. The ammonium salt represented by the Chemical Formula (XII) is particularly preferably benzethonium chloride.

Note that benzethonium chloride (Bzn⁺Cl⁻) can be represented by the chemical formula below. Also, benzalkonium chloride can be represented by Chemical Formula (XIII) above as a compound in which R¹¹¹ is an alkyl group having 8 to 18 carbon atoms, and X⁻ is a chloride ion.

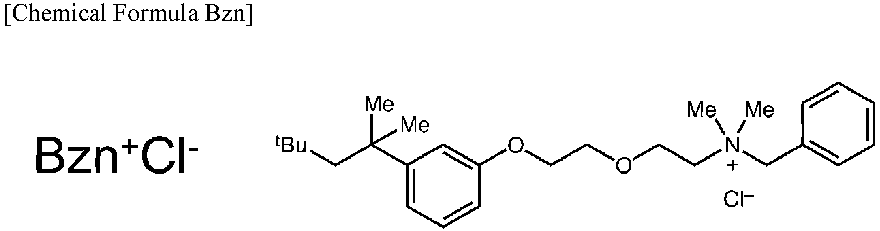

Note that, in the Chemical Formulae (XI), (XII), (XIII), (XIV), (XV), (XVI), and (XVII), X⁻ is any anion, and is not particularly limited. Also, X⁻ is not limited to a monovalent anion, and may be an anion having any valence, such as a divalent anion or a trivalent anion. When the anion is a polyvalent anion such as a divalent anion or a trivalent anion, the number of ammonium (monovalent) molecules in Chemical Formulae (XI), (XII), (XIII), (XIV), (XV), (XVI), and (XVII) is a value obtained by multiplying the number of anion molecules by the valence of the anions (for example, when the anion is a divalent anion, the number of ammonium (monovalent) molecules is twice as large as the number of anion molecules). Examples of X⁻ include halogen ions (a fluoride ion, a chloride ion, a bromide ion, and an iodide ion), an acetate ion, a nitrate ion, a sulfide ion, and a hexafluorophosphate ion.

In the radical generating catalyst according to the present invention, the ammonium salt may be, for example, a hexafluorophosphate salt of the above-mentioned ammonium.

In the present invention, the radical generating catalyst is not limited to Chemical Formulae (XI), (XII), (XIII), (XIV), (XV), (XVI), and (XVII) above, and may be, for example, an ammonium having any structure that includes an aromatic ring. Examples of the aromatic ring include, but are not limited to, aromatic rings listed as examples for R¹¹, R²¹, R³¹, and R⁴¹ in Chemical Formula (XI) above.

In the present invention, the radical generating catalyst may be, for example, a sulfonic acid-based amine or an ammonium thereof. The sulfonic acid-based amine is, for example, an amine having a sulfo group (sulfonate group) in its molecule. Examples of the sulfonic acid-based amine include taurine, sulfamic acid, 3-amino-4-hydroxy-1-naphthalenesulfonic acid, sulfamic acid, p-toluidine-2-sulfonic acid, o-anisidine-5-sulfonic acid, Direct Blue 14, 3-[N,N-bis(2-hydroxyethyl)amino]-2-hydroxypropanesulfonic acid, 3-[(3-cholamidopropyl)dimethylammonio]-1-propanesulfonate, aminomethanesulfonic acid, 3-sulfopropylamine, 2-aminobenzenesulfonic acid, R(+)-3-aminotetrahydrofuran toluene, 4-amino-5-hydroxy-1,7-naphthalenedisulfonic acid, N-(2-acetamide)-2-aminoethanesulfonic acid, sodium 4'-amino-3'-methoxyazobenzene-3-sulfonate, Lapatinib ditosylate, N-tris(hydroxymethyl)methyl-2-aminoethanesulfonic acid, disodium 8-amino-1,3,6-naphthalenetrisulfonate hydrate, 1-aminonaphthalene-2-sulfonic acid, (2S,3S)-3-amino-2-methyl-4-oxo-1-azetidinesulfonic acid, sodium 3-(1-naphthylamino)propanesulfonate, 3-methyl-4-aminobenzenesulfonic acid, sodium 3-cyclohexylamino-2-hydroxypropanesulfonate, sodium N-tris(hydroxymethyl)methyl-2-aminoethanesulfonate, 4-amino-1-naphthalenesulfonic acid, sodium sulfamate, tricaine, sodium sulfanilate, 1,4-phenylenediamine-2-sulfonic acid, p-anisidine-2-sulfonic acid, 6-amino-1-naphthalenesulfonic acid, 3,4-diaminobenzenesulfonic acid, 3-amino-4-chlorobenzenesulfonic acid, 3-[(4-amino-3-methylphenyl)azo]benzenesulfonic acid, 3-amino-4-hydroxy-5-nitrobenzenesulfonic acid, 5-amino-6-hydroxy-3-nitrobenzenesulfonic acid, 4-acetamide-2-aminobenzenesulfonic acid hydrate, 2-aminophenol-4-sulfonic acid, 1-amino-2-methoxy-5-methyl-4-benzenesulfonic acid, dansyl acid, Sulfamic acid [(1S,2S,4R)-4-[4-[[(1S)-2,3-dihydro-1H-inden-1-yl]amino]-7H-pyrrolo[2,3-d]pyrimidin-7-yl]-2-hydroxycyclopentyl]methyl ester, 5-sulfo-4'-diethylamino-2,2'-dihydroxyazobenzene, 2-aminonaphthalene-6,8-disulfonic acid, sodium 2-[N,N-bis(2-hydroxyethyl)amino]-1-ethanesufonate, 3-acetyl-2-(methylaminosulfonyl)thiophene, sodium 4-amino-2-chlorotoluene-5-sufonate, 5-(3-AMINO-5-OXO-2-PYRAZOLIN-1-YL)-2-PHENOXYBENZENESULFONIC ACID, potassium sulfamate, P-AMINOAZOBENZENE MONOSULFONIC ACID, 3-[(3-Cholamidopropyl)dimethylammonio]-2-hydroxy-1-propanesulfonate, sodium 3-amino-2,7-naphthalenedisulfonate, sodium 3-[N,N-bis(hydroxyethyl)amino]-2-hydroxypropanesulfonate, cobalt (II) di(amidosulfate), 3-(4-amino-3-methoxyphenylazo)benzenesulfonic acid, nickel (II) sulfamate tetrahydrate, 2,4-diaminobenzenesufonate sodium , 5-amino-2-chlorotoluene-4-sulfonic acid, 2,5-dichlorosulfanilic acid, 4-methylbenzenesulfonic acid, APTS (aminopyrenetrisulfonic acid), 4'-aminoazobenzene-3-sulfonic acid, pontacyl carmine 2B, p-anisidine-3-sulfonic acid, 4,4'-bis(4-amino-1-naphthylazo)-2,2'-stilbenesulfonic acid, 3-AMINONAPHTHALENE-8-HYDROXY-4,6-DISULFONIC ACID, sodium 4-amino-1,5-naphthalenedisufonate, sodium 4-aminoazobenzene-4'-sufonate, 5-amino-2-methylbenzenesulfonic acid, disodium 7-amino-1,3-naphthalenedisulfonate, alizarin safirol SE, sodium 7-amino-2-naphthalenesulfonate, 6-amino-5-bromopyridine-3-sulfonic acid, 2-aminoethanethiol p-toluenesulfonate, sodium 2-amino-1-naphthalenesufonate, disodium 6-amino-1,3-naphthalenedisulfonate hydrate, N,N,N',N'-tetraethylsulfamide, 5-amino-2-ethoxybenzenesulfonic acid, 3,5-diamino-2,4,6-trimethylbenzenesulfonic acid, 7-amino-1-naphthalenesulfonic acid, guanidine sulfamate, 2-amino-5-nitrobenzenesulfonic acid, nickel (II) diamidosulfate, disodium 4-amino-4'-nitrostilbene-2,2'-disulfonate, sodium aniline-2,5-disulfonate, 5-amino-1-naphthol-3-sulfonic acid hydrate, sodium 2,5-dichlorosulfanilate, hexyl 6-aminohexanoate p-toluenesulfonate, rac-(R^{∗})-2-(4-chlorophenyl)-3-amino-1-propanesulfonic acid, 2-(N,N-dipropyl)amino anisole-4-sulfonic acid, 2-amino-4-chlorophenol-6-sulfonic acid, 6-amino-1,3-naphthalenedisulfonic acid, 5,10,15,20-tetrakis[4-(trimethylammonio)phenyl]-21H,-23H-porphinetetratosylate, 5-amino-2-[(4-aminophenyl)amino]benzenesulfonic acid, 4-amino-3-chlorobenzenesulfonic acid, 2-aminobenzenesulfonic acid phenyl ester, disodium 4-acetylamino-4'-isothiocyanatostilbene-2,2'-disulfonate, (S)-3-AMINO-2-OXETANONE P-TOLUENESULFONIC ACID SALT, disodium 5-acetylamino-4-hydroxy-2,7-naphthalenedisulfonate, 2-phenylamino-5-aminobenzenesulfonic acid, sodium 4-octadecylamino-4-oxo-2-[(sodiooxy)sulfonyl]butanoate, and 3,5-diamino-4-methylbenzenesulfonic acid.

In the present invention, the radical generating catalyst may be, for example, a nicotine-based amine or an ammonium thereof. The nicotine-based amine is, for example, an amine having, in the molecule, a ring structure that includes a nicotine skeleton. Examples of the nicotine-based amine include nicotinamides and alkaloids.

In the present invention, the radical generating catalyst may be, for example, a nitrous acid-based amine or a nitrous acid-based ammonium. The nitrous acid-based amine or nitrous acid-based ammonium is a compound obtained through, for example, a reaction of an amine with nitrous acid or a nitrous acid derivative. Examples of the nitrous acid-based amine or nitrous acid-based ammonium include diazo compounds, diazonium salts, N-nitroso compounds, and C-nitroso compounds.

In the radical generating catalyst according to the present invention, the ammonium may include a plurality of ammonium structures (N⁺) in a single molecule. Furthermore, a plurality of the ammonium molecules may be associated due to π electron interaction to form a dimer, trimer, or the like.

In the radical generating catalyst according to the present invention, the amino acid is not particularly limited. It is sufficient that the amino acid includes, for example, at least one amino group or imino group and at least one carboxy group in the molecule. The amino acid may be, for example, an α-amino acid, a β-amino acid, a γ-amino acid, or an amino acid other than these amino acids. The amino acid may be, for example, an amino acid included in a protein. Specifically, the amino acid may be, for example, at least one selected from the group consisting of glycine, alanine, valine, leucine, isoleucine, serine, threonine, aspartic acid, glutamic acid, asparagine, glutamine, lysine, hydroxylysine, arginine, cysteine, cystine, methionine, phenylalanine, tyrosine, tryptophan, histidine, proline, and 4-hydroxyproline.

In the present invention, the peptide is not particularly limited. It is sufficient that the peptide is constituted by two or more of the above-mentioned amino acids that link together via peptide bonds. The peptide may be, for example, at least one of oxidized glutathione (GSSG) and reduced glutathione (GSH).

In the present invention, the phospholipid is not particularly limited. It is sufficient that the phospholipid is, for example, a lipid that includes a phosphorus atom in the molecule. The phospholipid may be, for example, a lipid that includes a phosphoester bond (P-O-C) in the molecule. The phospholipid optionally includes, for example, at least one of an amino group, an imino group, an ammonium group, and an iminium group in the molecule. The phospholipid may be, for example, at least one selected from the group consisting of phosphatidyl serine, phosphatidyl choline, phosphatidic acid, phosphatidylethanolamine, phosphatidyl glycerol, and cardiolipin.

The radical generating catalyst according to the present invention may include, for example, a Brønsted acid. The acid dissociation constant pKₐ of the Brønsted acid is, for example, 5 or more. The maximum value of the pKₐ above is not particularly limited, and is, for example, 50 or less.

The radical generating catalyst according to the present invention may, for example, catalyze the generation of a radical from the radical generation source in a non-acidic reaction system or catalyze the generation of a radical from the radical generation source in an acidic reaction system. Also, the radical generating catalyst according to the present invention may catalyze, for example, the generation of a radical from the radical generation source in a solution.

The radical generating catalyst according to the present invention may, for example, catalyze the generation of a radical from the radical generation source in vitro, and may also catalyze the generation of a radical from the radical generation source in vivo. The term "in vivo" may mean, for example, "in a human body", but may also mean "in the body of an animal other than a human".

Also, the radical generating catalyst according to the present invention may catalyze, for example, the generation of a radical from the radical generation source in a digestive organ. The digestive organ may be, for example, at least one selected from the group consisting of an oral cavity, a pharynx, an esophagus, a stomach, a duodenum, a small intestine, and a large intestine. The digestive organ may be, for example, a large intestine. The small intestine may be, for example, at least one selected from the group consisting of a duodenum, a jejunum, and an ileum. The large intestine may be, for example, at least one selected from the group consisting of a cecum, a colon, and a rectum. The radical generating catalyst according to the present invention may be used to, for example, disinfect the inside of the digestive organ, induce changes in the intestinal bacterial flora, treat ulcerative colitis, or suppress symptoms of ulcerative colitis.

The radical generating catalyst according to the present invention includes, for example, an ammonium salt (other than peroxodisulfate) represented by Chemical Formula (XI) above whose Lewis acidity is 0.4 eV or more, and catalyzes, in a non-acidic solution, the generation of a radical from a radical generation source that may be at least one selected from the group consisting of a halous acid, a halous acid ion, and a halous acid salt.

Note that, when isomers such as tautomers or stereoisomers (e.g., geometrical isomers, conformational isomers, and optical isomers) for the compound (e.g., the above-mentioned ammonium, amino acid, protein, peptide, or phospholipid) used in the present invention are present, all the isomers can be used in the present invention unless otherwise stated. When the compound can form a salt, this salt can also be used in the present invention unless otherwise stated. The salt may be an acid addition salt, and may also be a base addition salt. Furthermore, an acid included in the acid addition salt may be an inorganic acid or an organic acid, and a base included in the base addition salt may be an inorganic base or an organic base. Examples of the inorganic acid include, but are not particularly limited to, sulfuric acid, phosphoric acid, hydrofluoric acid, hydrochloric acid, hydrobromic acid, hydroiodic acid, hypofluorous acid, hypochlorous acid, hypobromous acid, hypoiodous acid, fluorous acid, chlorous acid, bromous acid, iodous acid, fluoric acid, chloric acid, bromic acid, iodic acid, perfluoric acid, perchloric acid, perbromic acid, and periodic acid. Examples of the organic acid include, but are also not particularly limited to, p-toluenesulfonic acid, methanesulfonic acid, oxalic acid, p-bromobenzenesulfonic acid, carbonic acid, succinic acid, citric acid, benzoic acid, and acetic acid. Examples of the inorganic base include, but are not particularly limited to, ammonium hydroxide, alkali metal hydroxides, alkaline earth metal hydroxides, carbonates and hydrogencarbonates. More specific examples thereof include sodium hydroxide, potassium hydroxide, potassium carbonate, sodium carbonate, sodium bicarbonate, potassium bicarbonate, calcium hydroxide, and calcium carbonate. Examples of the organic base include, but are also not particularly limited to, ethanolamine, triethylamine, and tris(hydroxymethyl)aminomethane. There is no particular limitation on a method for manufacturing these salts, and these salts can be manufactured, for example, using a method in which an acid or base as mentioned above is added to the compound as appropriate using a known technique.

In the present invention, a chain substituent (e.g., a hydrocarbon group such as an alkyl group or unsaturated aliphatic hydrocarbon group) may be a linear substituent or branched substituent unless otherwise stated, and may have, for example, 1 to 40, 1 to 32, 1 to 24, 1 to 18, 1 to 12, 1 to 6, or 1 to 2 carbon atoms (an unsaturated hydrocarbon group has two or more carbon atoms). Also, in the present invention, the number of ring members (the numer of atoms included in a ring) in a cyclic group (e.g., an aryl group or heteroaryl group) is not particularly limited, and may be, for example, 5 to 32, 5 to 24, 6 to 18, 6 to 12, or 6 to 10. When isomers for the substituent or the like are present, any isomer can be used unless otherwise stated. For example, a reference merely to a "naphthyl group" may mean a 1-naphthyl group or a 2-naphthyl group.

### 1-2. Radical Generation Source

In the drug according to the present invention, the radical generation source may include, for example, at least one selected from the group consisting of a halogen ion, a hypohalous acid ion, a halous acid ion, a halogen acid ion, and a perhalogen acid ion. It is particularly preferable that the radical generation source includes, for example, chlorous acid ions. The radical generation source may include, for example, at least one selected from the group consisting of an oxoacid, an oxoacid salt, and an oxoacid ion (e.g., a halogen oxoacid or salts thereof). Examples of the oxoacid include boric acid, carbonic acid, orthocarbonic acid, carboxylic acid, silicic acid, nitrous acid, nitric acid, phosphorous acid, phosphoric acid, arsenic acid, sulfurous acid, sulfuric acid, sulfonic acid, sulfinic acid, chromic acid, dichromic acid, permanganic acid, and a halogen oxoacid. Examples of the halogen oxoacid include: chlorine oxoacids such as hypochlorous acid, chlorous acid, chloric acid, and perchloric acid; bromine oxoacids such as hypobromous acid, bromous acid, bromic acid, and perbromic acid; and iodine oxoacids such as hypoiodous acid, iodous acid, iodic acid, and periodic acid.

The radical generation source may include, for example, at least one selected from the group consisting of a halogen ion, a hypohalous acid ion, a halous acid ion, a halogen acid ion, and a perhalogen acid ion. The radical generation source may be, for example, at least one selected from the group consisting of a halous acid, a halous acid ion, and a halous acid salt, or at least one selected from the group consisting of chlorous acid, bromous acid, and iodous acid, or a chlorous acid ion.

The radical generation source may be selected as appropriate, for example, depending on the intended use in consideration of the reactivity level of a radical species and the like. For example, hypochlorous acid, which is highly reactive, and chlorous acid, which has slightly milder reactivity than hypochlorous acid and has high reaction-controllability, may be used for different purposes.

### 1-3. Stabilizer

As described above, the drug according to the present invention may or may not include another component in addition to the radical generating catalyst and the radical generation source. The other component is not particularly limited and is optional. One type of component or a plurality of types of components may be included as the other component. It is preferable that the drug according to the present invention includes a stabilizer as the other component.

The stabilizer serves to, for example, suppress or prevent the degradation of the radical generation source in a solid drug according to the present invention. Specifically, the stabilizer may also be, for example, a substance that suppresses or prevents the generation of a radical from the radical generation source in the solid drug. When the degradation of the radical generation source is suppressed or prevented in the solid drug according to the present invention, the shelf life of the solid drug is improved when the solid drug is transported or stored as it is.

For example, it is preferable that the drug according to the present invention is configured as follows: the degradation of the radical generation source (e.g., the generation of a radical from the radical generation source) is suppressed or prevented in a solid drug, and a radical is generated from the radical generation source after the solid drug is dissolved or dispersed in a liquid medium to produce a liquid drug.

The stabilizer to be used in the drug according to the present invention may be, for example, a substance that can oxidize or reduce a radical generated from the radical generation source. This makes it possible to suppress or prevent the discharge (release) of the generated radical.

The stabilizer may be at least one of a carbonate and a hydrogencarbonate. Examples of the carbonate include alkali metal carbonates and alkali earth metal carbonates. For example, sodium carbonate, potassium carbonate, calcium carbonate, barium carbonate, magnesium carbonate, aluminum carbonate, lithium carbonate, ammonium carbonate, and the like can be used. Examples of the hydrogencarbonate include alkali metal hydrogencarbonates and alkali earth metal hydrogencarbonates. For example, sodium hydrogencarbonate, potassium hydrogencarbonate, calcium hydrogencarbonate, barium hydrogencarbonate, magnesium carbonate, aluminum carbonate, lithium carbonate, ammonium carbonate, and the like can be used.

In an example described below, a chlorous acid ion (ClO₂⁻) is used as the radical generation source, and sodium carbonate is used as the stabilizer.

The concentrations of components in a solid drug are much higher than those in a liquid drug obtained by dissolving or dispersing the solid drug in a medium (e.g., water). The concentrations of components in the solid drug may be, for example, about 1000 times as high as those in the liquid drug in terms of a weight ratio. Accordingly, reactions represented by Chemical Reaction Formulae (1) and (2) may occur in the solid drug under the influence of light, moisture, or the like. In these reactions, a chlorous acid ion (ClO₂⁻) serving as the radical generation source is degraded, and thus hydrogen peroxide (H₂O₂) is generated.

### [Formulae 1 and 2]

ClO₂^{·} + 2ClO₂⁻ → 2ClO₂⁻ + Cl⁻ + O₂^{·-} (1)

2O₂^{·-} + 2H⁺ → H₂O₂ + O₂ (2)

The reactions represented by Chemical Reaction Formulae (1) and (2) can be suppressed or prevented by blending sodium carbonate in the solid drug. Specifically, it is conceivable that a carbonate ion (CO₃²⁻) derived from sodium carbonate reacts with hydrogen peroxide and a chlorine dioxide radical (ClO₂^{·}) as represented by Chemical Reaction Formula (3) below. As a result, as represented by Chemical Reaction Formula (3) below, the chlorous acid ion (ClO₂⁻) is returned to a chlorous acid ion, and the hydrogen peroxide is converted to oxygen (O₂). It is conceivable that the reactions represented by Chemical Reaction Formulae (1) and (2) above are thus suppressed or prevented. Accordingly, the generation of a chlorine dioxide radical caused by the degradation of a chlorous acid ion is suppressed or prevented, and thus the solid drug is stabilized.

### [Formula 3]

2CO₃²⁻ + H₂O₂ + 2ClO₂^{·} ↔ 2ClO₂⁻ + O₂ + 2HCO₃⁻ (3)

Note that, if a stabilizer is blended in the solid drug, it is possible to suppress excessively rapid generation of a radical from the radical generation source and enhance the stability and safety even when the solid drug is, for example, dissolved or dispersed in a medium (e.g., water) to produce a liquid drug.

Also, even if, for example, hydrogen peroxide is generated in the liquid drug due to degradation of a chlorous acid ion, the concentration of the hydrogen peroxide is generally too low to cause a safety problem. However, if a stabilizer (e.g., sodium carbonate) is blended in the drug, it is possible to suppress or prevent the generation of hydrogen peroxide due to the degradation of a chlorous acid ion and further enhance the stability and safety.

### 1-4. Other Components

As described above, the drug according to the present invention may or may not include another component in addition to the radical generating catalyst and the radical generation source. In the drug according to the present invention, the other component is not limited to the stabilizer. The drug according to the present invention may or may not include the stabilizer, and may or may not include a component other than the stabilizer as the other component.

Examples of the other component include liquid property-controlling (acidity/basicity-controlling) agents and buffers in addition to the stabilizers. The liquid property-controlling agents prepare, for example, the liquid property of a liquid drug obtained by dissolving or dispersing the drug according to the present invention in a medium (e.g., water). For example, blending the liquid property-controlling agent makes it possible to prevent the liquid drug from being made acidic, and thus suppress or prevent excessively rapid degradation of the radical generation agent (excessively rapid generation of a radical). Thus, the stability and safety of the liquid drug can be enhanced. Examples of the liquid property-controlling agent include, but are not particularly limited to, carbonates, hydrogencarbonates, phosphates, hydrogenphosphates, sodium citrate, and citric acid. These agents may be used alone or in combinations of two or more. Examples of the above-mentioned carbonates and hydrogencarbonates include the carbonates and hydrogencarbonates listed as the examples of the stabilizer. Examples of the phosphates include sodium phosphate (Na₃PO₄) and potassium phosphate. Examples of the hydrogenphosphates include disodium hydrogenphosphate (Na₂HPO₄), sodium dihydrogenphosphate (NaH₂PO₄), dipotassium hydrogenphosphate (K₂HPO₄), and potassium dihydrogenphosphate (KH₂PO₄). For example, the stabilizer may also serve as a liquid property-controlling agent. The liquid property-controlling agent may be, for example, a pH adjuster. Examples of the buffers include, but are not limited to, the hydrogenphosphates and dihydrogenphosphates listed as the examples of the liquid property-controlling agent. For example, the buffer may also serve as a liquid property-controlling agent.

### 1-5. Content Ratios of Components

The content ratios of the components in the solid drug according to the present invention are not particularly limited and can be set as appropriate. As described above, the solid drug according to the present invention may or may not include the radical generating catalyst. Also, as described above, the drug according to the present invention may or may not include the components (e.g., the stabilizer, the liquid property-controlling agent, and the buffer) other than the radical generation source and the radical generating catalyst.

### 2. Drug Manufacturing Method

A method for manufacturing the solid drug according to the present invention is not particularly limited. For example, the solid drug can be manufactured using the above-described drug manufacturing method according to the present invention. As described above, the drug manufacturing method according to the present invention is characterized by including a pressing step of pressing a composition containing the constituent components of the drug according to the present invention into a solid form.

There is no particular limitation on the pressure and the pressing time in the pressing step. It is preferable that the pressure in the pressing step is not excessively low in order to suppress or prevent disintegration of the solid drug during transportation and storage. Also, it is preferable that the pressure in the pressing step is not excessively high in order to facilitate dissolution or dispersion of the solid drug according to the present invention in a medium (e.g., water).

The drug manufacturing method according to the present invention may or may not include any step in addition to the pressing step. For example, the drug manufacturing method according to the present invention may include a mixing step of mixing the constituent components of the drug according to the present invention prior to the pressing step. In the drug manufacturing method according to the present invention, it is preferable that the constituent components of the drug according to the present invention are mixed as uniformly as possible, and then the pressing step is performed to press the obtained mixture. For example, the constituent components of the drug according to the present invention may be processed into minute particles, powder, or the like prior to the mixing step. The size of the minute particles or powder is not particularly limited, and can be set as appropriate.

For example, the surface of the solid drug according to the present invention may be coated, or the solid drug according to the present invention may be encapsulated in a microcapsule, in order to suppress or prevent the degradation caused by moisture absorption or the like. These methods are not particularly limited, and may be performed, for example, in conformity with methods used for common drugs.

### 3. Drug Usage

As described above, the drug according to the present invention can be dissolved or dispersed in a liquid medium before use. The drug according to the present invention is a solid drug (e.g., tablet) and is thus very convenient to transport and store as described above. For example, adding the stabilizer to the drug further improves the stability of the drug. When the drug according to the present invention is used, a very convenient drug that can be used for spraying (atomizing), application, and the like can be obtained through a step of producing a liquid drug by dissolving or dispersing a solid drug in a liquid medium. The mass (weight) of the liquid medium is not particularly limited, but it is sufficient that the mass (weight) is determined as appropriate in consideration of, for example, the mass ratio (weight ratio) of the solid drug according to the present invention to the total mass (total weight). For example, it is sufficient that the mass ratio of the liquid medium to the mass of the solid drug according to the present invention is reduced in order to increase the concentration of the radical generation source in the liquid drug. Also, if, for example, the content of the radical generation source in the solid drug according to the present invention is small due to the content of components other than the radical generation source being large, the mass ratio of the liquid medium to the mass of the solid drug according to the present invention may be set to be small in order to increase the concentration of the radical generation source in the liquid drug. For example, the concentration of the radical generation source may be the same as that in the liquid drug described in Patent Literature 1, but this is merely exemplary, and the present invention is not limited to only this example.

The liquid medium may be, for example, at least one of water and an organic solvent, and may be water. There is no particular limitation on the liquid property of a solution or dispersion liquid obtained by dissolving or dispersing the drug according to the present invention in the liquid medium, but it is preferable that the solution or dispersion liquid is non-acidic. As described above, when the obtained solution or dispersion liquid is non-acidic, excessively rapid degradation of the radical generation agent (excessively rapid generation of a radical) is suppressed or prevented, thus making it possible to enhance the stability and safety of the drug.

The following is a description of mainly a case where the solid drug according to the present invention is dissolved or dispersed in a liquid medium and is used as a liquid drug. In particular, a case where the solid drug according to the present invention is dissolved in water to produce an aqueous solution will be mainly described. In the following description, a liquid drug manufactured by dissolving or dispersing the solid drug according to the present invention in a liquid medium may also be referred to as a "liquid drug according to the present invention".

With the present invention, it is possible to provide a drug that is highly safe and highly disinfectant.

The application of the drug according to the present invention is not particularly limited. The drug according to the present invention can be used for a wide variety of applications as described below. The drug according to the present invention can be used in, for example, the above-described water purification method according to the present invention. As described above, the water purification method according to the present invention is the water purification method that includes a step of adding the drug according to the present invention, or a drug-containing solution containing the drug according to the present invention, into water. The "drug-containing solution" containing the drug according to the present invention is not particularly limited, and may be, for example, the above-described liquid drug produced by dissolving or dispersing the solid drug according to the present invention in a liquid medium. The water purification may include, for example, at least one of disinfection and deodorization of the water. The water to be purified is not particularly limited, and may be, for example, water of the ocean, river, pond, and the like.

Also, the drug according to the present invention can be used as, for example, a drug for agriculture and stockbreeding. In the following description, the drug according to the present invention capable of being used as a drug for agriculture and livestock may also be referred to as a "drug for agriculture and stockbreeding according to the present invention".

The drug for agriculture and stockbreeding according to the present invention is highly safe and highly disinfectant. Accordingly, the drug for agriculture and stockbreeding according to the present invention can be used, for example, in a wide variety of applications such as disinfection and deodorization in the agricultural and livestock industries. Also, the drug for agriculture and stockbreeding according to the present invention is unlikely to, for example, cause a corrosion, and is unlikely to corrode metals even when applied to metals. Accordingly, the drug for agriculture and stockbreeding according to the present invention can be used for, for example, a metal-containing target.

In the drug according to the present invention, for example, the Lewis acidity of the radical generating catalyst according to the present invention is not particularly limited, and is, for example, 0.4 eV or more, 0.5 eV or more, or 0.6 eV or more, and, for example, 20 eV or less, as described above.

The liquid drug according to the present invention may be acidic or non-acidic, or basic or non-basic, or neutral or non-neutral, but is preferably non-acidic.

The liquid drug according to the present invention may be, for example, a liquid drug that includes:
the radical generating catalyst according to the present invention; and at least one selected from the group consisting of a halous acid, a halous acid ion, and a halous acid salt,
wherein the radical generating catalyst according to the present invention is a radical generating catalyst that catalyzes the generation of a radical from at least one radical generation source selected from the group consisting of a halous acid, a halous acid ion, and a halous acid salt, the Lewis acidity of the radical generating catalyst being 0.4 eV or more, and
the liquid drug is non-acidic.

In the liquid drug according to the present invention, the radical generation source may be selected as appropriate, for example, depending on the intended use in consideration of the reactivity level of a radical species and the like. For example, hypochlorous acid, which is highly reactive, and chlorous acid, which has slightly milder reactivity than hypochlorous acid and has high reaction-controllability, may be used for different purposes.

The content of the radical generation source (e.g., an oxoacid) in the liquid drug according to the present invention is not particularly limited, and is, for example, 0.01 mass ppm or more, 0.05 mass ppm or more, or 0.1 mass ppm or more, and 1500 mass ppm or less, 1000 mass ppm or less, or 250 mass ppm or less. The concentration of the radical generation source (e.g., an oxoacid) mixed in the drug is preferably 0.01 to 1500 mass ppm, more preferably 0.05 to 1000 mass ppm, and even more preferably 0.1 to 250 mass ppm. It is conceivable that the lower the concentration is, the safer the drug is. Therefore, a lower concentration is preferable. However, if the concentration is excessively low, there is a risk that a disinfective effect and the like cannot be obtained. From the viewpoint of the disinfective effect, there is no particular limitation on the concentration of the radical generation source, and the higher the concentration is, the better it is.

The content of the radical generating catalyst (e.g., an ammonium or cationic surfactant) in the liquid drug according to the present invention is not particularly limited, and is, for example, 0.01 mass ppm or more, 0.05 mass ppm or more, or 0.1 mass ppm or more, and 1500 mass ppm or less, 1000 mass ppm or less, 500 mass ppm or less, or 250 mass ppm or less. The concentration of the radical generating catalyst (e.g., an ammonium or cationic surfactant) mixed in the drug is preferably 0.01 to 1500 mass ppm, more preferably 0.05 to 1000 mass ppm, even more preferably 0.05 to 500 mass ppm, and even more preferably 0.1 to 250 mass ppm. It is conceivable that the lower the concentration is, the safer the drug is. Therefore, a lower concentration is preferable. However, if the concentration is excessively low, there is a risk that a disinfective effect and the like cannot be obtained. Also, the concentration of the radical generating catalyst is preferably lower than or equal to the critical micelle concentration from the viewpoint of preventing failure to obtain a disinfective effect and the like due to micelle formation.

The ratio between the concentrations of the radical generation source and the radical generating catalyst (radical generation source / radical generating catalyst) in the liquid drug according to the present invention is not particularly limited, and can be set as appropriate.

The liquid drug according to the present invention may further include another substance. Examples of the other substance include water, an organic solvent, the stabilizer, the liquid property-controlling agent (e.g., pH adjuster), and the buffer, and these substances may be used alone or in combination of two or more (the same applies to the following). The water is not particularly limited, but is preferably purified water, ion-exchanged water, or pure water.

It is preferable that the liquid drug according to the present invention includes at least one of water and an organic solvent. Note that the radical generating catalyst according to the present invention, the radical generation source according to the present invention, and the like may be soluble or insoluble in the "solvent" used in the present invention. For example, after the mixing step, the radical generating catalyst and the radical generation source according to the present invention may be dissolved in the solvent, or may be dispersed or precipitated in the solvent. Also, it is preferable to use water in the drug according to the present invention as a solvent for the radical generating catalyst and the radical generation source according to the present invention from the viewpoint of the safety, the cost, and the like. Examples of the organic solvent include ketones such as acetone, nitrile solvents such as acetonitrile, and alcohol solvents such as ethanol, and these solvents may be used alone or in combination of two or more. Different types of solvents may be used depending on, for example, the solubility of a solute (e.g., the radical generating catalyst and the radical generation source according to the present invention) and the like.

The pH of the liquid drug according to the present invention is not particularly limited, but may be, for example, 4.0 or more, 4.5 or more, 5.0 or more, 5.5 or more, 6.0 or more, 6.5 or more, 7.0 or more, or 7.5 or more. Also, the pH of the drug according to the present invention may be, for example, 11.5 or less, 11.0 or less, 10.5 or less, 10.0 or less, 9.5 or less, 9.0 or less, 8.5 or less, 8.0 or less, or 7.5 or less.

The liquid drug according to the present invention can be manufactured by, for example, dissolving or dispersing the solid drug according to the present invention in a medium as described above. For example, the liquid drug according to the present invention can be obtained in the same manner as described in Examples, which will be described later, but there is no limitation thereto. Also, another substance other than the radical generation source and the radical generating catalyst may be mixed in the liquid drug according to the present invention as described above. The other substance may be, for example, the other substance included in the solid drug according to the present invention, other than the radical generating catalyst and the radical generation source.

The drug for agriculture and stockbreeding according to the present invention preferably includes, for example, the above-mentioned water, but may also include no water. The amount of the water mixed in the drug for agriculture and stockbreeding (water ratio) is not particularly limited. The water ratio may correspond to, for example, a portion other than the other components. The drug for agriculture and stockbreeding may or may not include, for example, the pH adjuster, the buffer, and the like as the other substances.

There is no particular limitation on the usage of the liquid drug according to the present invention, and the liquid drug can be used, for example, as in the case of a conventional disinfectant and the like. Specifically, the liquid drug according to the present invention may be sprayed or applied onto a target, for example. Specifically, the liquid drug can be sprayed in order to, for example, deodorize a space. An aqueous solution can be used for gargling or washing inside the oral cavity. When used to sterilize a bedsore, the liquid drug can be applied onto the affected site. In the case of a fungating cancer wound or a site affected by Trichophyton and the like, absorbent cotton or gauze impregnated with the liquid drug can be attached to the affected site. When the liquid drug is used for hand sterilization, an aqueous solution thereof can be rubbed onto hands. Medical devices and the like can be washed with the liquid drug by spraying the liquid drug thereonto or immersing them into an aqueous solution of the liquid drug. The liquid drug can be applied onto a bed and its surroundings, a table and its surroundings, a doorknob, and the like for the purpose of disinfection and prevention.

### Disinfectant

The drug according to the present invention can be used as, for example, a disinfectant. Conventionally, a variety of disinfectants have been used, but their disinfective effects are not satisfactory. Although the disinfective effects can be improved by increasing the concentrations thereof in some cases, safety problems may arise. A disinfectant that includes the drug according to the present invention exhibits sufficient disinfective effects even at a low concentration, and is thus highly safe.

### Disinfectant for Hand Sterilization

The drug according to the present invention can be used as, for example, a disinfectant for hand sterilization to be used to sterilize hands, fingers and the like. A disinfectant for hand sterilization that includes the drug according to the present invention exhibits sufficient disinfective effects even at a low concentration, and is thus highly safe.

### Deodorant

The drug according to the present invention can be used as, for example, a deodorant. Commonly used disinfectants such as ethanol have no deodorizing effects. Chlorine dioxide has deodorizing effects, but is extremely low in safety. Some of other commercially available products are described as having disinfective effects and deodorizing effects. For example, there are products purporting to exhibit disinfective effects and deodorizing effects by spraying a drug directly onto clothes or in rooms, rest rooms, or vehicles. In general, a quaternary ammonium salt is used as a disinfectant component in such products. However, a commonly used quaternary ammonium salt is not used together with a radical generation source (e.g., oxoacid). Accordingly, in many cases, sufficient disinfective effects are not exhibited unless it is used at a high concentration, and a problem of stickiness may arise after use. Also, a quaternary ammonium salt has no deodorizing effects, and thus a deodorizing component is mixed separately. Cyclodextrin is generally used as the deodorizing component. However, cyclodextrin has no ability to decompose a component responsible for unpleasant odors, and merely masks the component responsible for unpleasant odors, that is, cyclodextrin cannot remove unpleasant odors. In contrast, a deodorant that includes the drug according to the present invention has the above-mentioned mechanism of action. Accordingly, this deodorant has, for example, high disinfective effects. In addition, the deodorant can, for example, decompose a substance responsible for unpleasant odors, and thus has high deodorizing effects.

### Antibacterial Agent for Metals

The drug according to the present invention can be used as, for example, an antibacterial agent for metals. An antibacterial agent that includes the drug according to the present invention is highly safe, and thus can be sprayed or applied onto, for example, metal products to be used in the kitchen. Also, an antibacterial agent that includes the drug according to the present invention is unlikely to cause a corrosion, and is thus unlikely to corrode metals even when applied to metals.

### Oral Care Agent

The drug according to the present invention can be used as, for example, an oral care agent. An oral care agent that includes the drug according to the present invention is highly safe, and is thus suitable for use in the oral cavity.

### Acne Treatment Drug

The drug according to the present invention can be used as, for example, an acne treatment drug. An acne treatment drug that includes the drug according to the present invention is highly safe, and thus can be applied onto a face.

### Bedsore Sterilizing Agent

The drug according to the present invention can be used as, for example, a bedsore sterilizing agent. A bedsore sterilizing agent that includes the drug according to the present invention is highly safe, and thus can be applied onto a body.

### Disinfectant for Fungi

The drug according to the present invention can be used as, for example, a disinfectant for sterilizing a site affected by fungi such as Trichophyton.

### Water Purification Disinfectant

The drug according to the present invention can kill, for example, bacteria such as Legionella growing in pool water or bathwater. In addition, this drug does not corrode metals and generates no gases. Accordingly, a water purification disinfectant that includes the drug according to the present invention can be used safely.

Furthermore, the drug according to the present invention can be used for, for example, applications as follows. As described above, the drug according to the present invention is highly safe and highly disinfectant. Accordingly, the drug according to the present invention can exhibit, for example, deodorizing effects and the like. Therefore, the drug according to the present invention is useful for, for example, improving QOL (quality of life).

As described above, the drug according to the present invention is highly safe, and thus can also be used for a human body. More specifically, this drug can be used for, for example, applications as follows.
(1) Prevention and treatment of cystitis, alleviation of symptoms thereof, etc.
(2) Prevention and treatment of candidiasis, alleviation of symptoms thereof, etc. (including oral irrigation and vaginal irrigation)
(2) Use in eyedrops and eyewash (including use for diseases such as a sty)
(3) Use for ear irrigation and nasal irrigation (otitis media, otitis externa, paranasal sinusitis, etc.)
(4) Oral irrigation and PMTC (professional mechanical tooth cleaning). This includes oral irrigation and PMTC for preventing aspiration pneumonia, and oral irrigation and PMTC for improving QOL (quality of life) of the elderly or disabled.
(5) Peritoneal lavage (including treatment of peritonitis, peritoneal dissemination, etc.)
(6) Bowel irrigation
(7) Sterilization, washing, etc. of dermal tissues
(8) Hand sterilization and hand washing
(9) Sterilization, washing, and cleansing of an affected site (including wounds)
(10) Treatment of dermatitis such as atopic dermatitis (e.g., sterilization, washing, cleansing, etc. of an affected site)
(11) Sterilization and washing of a site affected by infection of bacteria, such as perionychia, folliculitis, carbuncle, and furuncle

Also, the drug according to the present invention can be used for, for example, overall measures taken for prevention and treatment of infectious diseases. Specific examples are as follows.
(1) Prevention of upper respiratory tract infection (including influenza, SARS, and MERS)
(2) Prevention of food poisoning (caused by noroviruses, salmonellae, etc.)
(3) Treatment of vomit
(4) Inactivation of hepatitis B viruses and hepatitis C viruses
(5) Prevention and treatment of ulcerative colitis, alleviation of symptoms thereof, etc.
(6) Measures against mold and fungi (that is, the drug can be used for measures other than those against bacteria and viruses)
(7) Prevention and treatment of stomatitis, alleviation of symptoms thereof, etc. (e.g., side effects caused by molecular target drugs)
(8) Care before and after a surgery (including cancer surgeries, etc.)
(9) Care of a site affected by cancer (including an oral cavity, mammography, a fungating wound, etc.)

Furthermore, the drug according to the present invention has disinfective effects and is highly safe, and can thus be used for, for example, the following applications.
(1) Washing of an artificial tooth
(2) Disinfection of a baby feeding bottle
(3) Disinfection (for preventing infection), deodorization, etc. of everyday items touched by a large number of unspecified people, such as a desk and a doorknob
(4) Disinfection, deodorization, etc. of public transportation facilities such as trains, airplanes, and buses
(5) Disinfection of overall environments in schools, kindergartens, and nursery schools (including disinfection of desks, doors, shelves, switches, toys such as blocks, etc. for preventing infection, etc.)
(6) Disinfection, etc. of medical devices (including washing and disinfection of the inner parts and pipes of gastrocameras, laparoscopes, dialyzers, etc.)
(7) Cleansing, cleaning, or washing of diagnostic devices such as X-ray imaging machines, CTs, and electrocardiogram monitors
(8) Disinfection of tools to be used in medical examinations and surgeries (including metal tools such as scalpels, resin tools, etc.)

Furthermore, the drug according to the present invention has a proteolytic function, and can thus be used for, for example, the following applications.
(1) Contact lens storage solution
(2) Glasses cleaning solution
(3) Ultrasonic cleaner
(4) Cleansing agent
(5) Cleansing and cleaning of glass (including windshields)

Furthermore, the drug according to the present invention has a deodorizing function, and can thus be used to, for example, deodorize odors as follows.
(1) Breath odor
(2) Body odor
(3) Feces odor
(4) Odors of a chemical substances, gas, etc. (including overall odors in factories such as chemical factories and food factories)
(5) Trash-associated odors
(6) Odors from raw garbage, waste-collection points, waste collection vehicles, recycling centers, and incinerator plants
(7) Sewage pipe-associated odors
(8) Odors from pipes and trapping facilities such as oil traps
(9) Cigarette odor
(10) Odors associated with medical care (including use for improvement of QOL of patients as well as deodorization of unpleasant odors from fungating wounds)
(11) Odors in operating rooms
(12) Odors in hospital rooms
(13) Environmental odors (feces odors, wastewater odors) from poultry houses, pig houses, and cowhouses
(14) Odors in meat centers (slaughterhouses)

The drug according to the present invention may be used, for example, ex vivo. As described above, the drug according to the present invention is highly safe, and thus may also be used, for example, in vivo. For example, the drug according to the present invention may be configured such that the radical generating catalyst catalyzes the generation of a radical from the radical generation source in vivo. The term "in vivo" may mean, for example, "in a human body", but may also mean "in the body of an animal other than a human".

The drug according to the present invention may be used, for example, in a digestive organ. For example, the drug according to the present invention may be configured such that the radical generating catalyst catalyzes the generation of a radical from the radical generation source in a digestive organ. The digestive organ may be, for example, at least one selected from the group consisting of an oral cavity, a pharynx, an esophagus, a stomach, a duodenum, a small intestine, and a large intestine. The digestive organ may be, for example, a large intestine. The small intestine may be, for example, at least one selected from the group consisting of a duodenum, a jejunum, and an ileum. The large intestine may be, for example, at least one selected from the group consisting of a cecum, a colon, and a rectum. The drug according to the present invention may be used to, for example, disinfect the inside of the digestive organ, induce changes in the intestinal bacterial flora, treat ulcerative colitis, or suppress symptoms of ulcerative colitis.

The drug according to the present invention may be sprayed using, for example, an atomizer, a humidifier, or the like. In this case, for example, in addition to the disinfective effects on a spraying target, a disinfective function and a deodorizing function for an atomizer, a humidifier, or the like can also be obtained. Also, for example, the drug according to the present invention is not limited to use for a human, and can also be used for an animal other than a human. Specifically, this drug can be used to, for example, deodorize an animal, and prevent infectious diseases such as avian influenza and swine influenza. Examples of uses for an animal other than a human include the same uses as those listed above as the uses for a human (including a human body). Furthermore, examples of uses for an animal other than a human include uses as a drug for agriculture and stockbreeding, which will be described below.

As described above, the drug for agriculture and stockbreeding according to the present invention is highly safe and highly disinfectant. Accordingly, the drug for agriculture and stockbreeding according to the present invention can be used as, for example, a drug for agriculture, a drug for stockbreeding, or the like. The drug for agriculture can be used as, for example, a disinfectant for agriculture, an antiviral agent for agriculture, a deodorant for agriculture, a pesticide for agriculture, a repellent for agriculture, a soil conditioner for agriculture, and the like. The drug for stockbreeding can be used as, for example, a disinfectant for stockbreeding, an antiviral agent for stockbreeding, a deodorant for stockbreeding, a pesticide for stockbreeding, a repellent for stockbreeding, a soil conditioner for stockbreeding, and the like. The drug for agriculture and stockbreeding may be used for, for example, a single application or two or more of applications.

Examples of the agriculture include rice farming and dry-field farming. Examples of products obtained by the dry-field farming include vegetables such as cucumbers, tomatoes, Welsh onions, Chinese cabbages, and soybeans, potatoes such as Irish potatoes, flowering plants such as artificial-light grown chrysanthemum, clematis, and Banksian rose, fruits such as strawberries, and fertilizers. Examples of products obtained by the stockbreeding include industrial animals such as cows, pigs, and chickens.

When used in the rice farming, the drug for agriculture and stockbreeding according to the present invention can be used as, for example, a disinfectant, a pesticide, a repellent, a soil conditioner, and the like. Specifically, using the drug for agriculture and stockbreeding, for example, during the immersion of seed rice makes it possible to prevent the generation of slime and reduce the frequency of a water changing operation. Also, using the drug for agriculture and stockbreeding, for example, in seed immersion, hastening of germination, and seeding makes it possible to prevent a rice blast disease, a sheath blight disease, rice false smut, rice bakanae disease, and the like. For example, sprinkling a rice field with the drug for agriculture and stockbreeding makes it possible to keep stinkbugs, other harmful insects, and the like away from rice plants. For example, sprinkling a rice field with the drug for agriculture and stockbreeding during preparation for transplanting young rice plants makes it possible to improve the soil.

When used in the dry-field farming, the drug for agriculture and stockbreeding according to the present invention can be used as, for example, a disinfectant, an antiviral agent, a soil conditioner, and the like. Specifically, spraying the drug for agriculture and stockbreeding onto, for example, cucumber leaves, tomato leaves, or strawberry leaves makes it possible to prevent powdery mildew, a mosaic disease, and the like. Spraying the drug for agriculture and stockbreeding onto, for example, tomato leaves makes it possible to prevent a gray mold disease, a leaf mold disease, and the like. Spraying the drug for agriculture and stockbreeding onto, for example, Welsh onion leaves makes it possible to prevent a red rust disease and the like. Spraying the drug for agriculture and stockbreeding onto, for example, Chinese cabbage leaves makes it possible to prevent a root-knot disease and the like. For example, sprinkling a potato field with the drug for agriculture and stockbreeding after cultivation using a farm tractor or the like and then cultivating the potato field again makes it possible to prevent damage from continuous cropping. Immersing, for example, a seed potato in the drug for agriculture and stockbreeding makes it possible to sterilize (disinfect) the seed potato. Spraying the drug for agriculture and stockbreeding onto potato leaves a plurality of times, for example, between when a potato begins to sprout and when potatoes are harvested makes it possible to prevent a scab disease and the like. Spraying the drug for agriculture and stockbreeding onto, for example, artificial-light grown chrysanthemum, clematis, and Banksian rose makes it possible to prevent powdery mildew and the like.

When used in the stockbreeding, the drug for agriculture and stockbreeding according to the present invention can be used as, for example, a disinfectant, a deodorant, and the like. Specifically, using the drug for agriculture and stockbreeding as, for example, a cow dipping agent makes it possible to prevent mastitis and the like. For example, using the drug for agriculture and stockbreeding for cattle hoof bathing, or applying this drug onto a site affected by a hoof disease makes it possible to prevent or treat a hoof disease and the like. Atomizing the drug for agriculture and stockbreeding onto, for example, a cow using an atomizer makes it possible to prevent respiratory diseases, a foot-and-mouth disease, and the like. Atomizing the drug for agriculture and stockbreeding onto, for example, cowhouses, pig houses, poultry houses, and the like using an atomizer makes it possible to deodorize them. Using the drug for agriculture and stockbreeding for, for example, eggs makes it possible to sterilize (disinfect) the eggs.

The drug for agriculture and stockbreeding according to the present invention may be, for example, sprayed (atomized), applied, or sprinkled onto the target. The target may be immersed in the drug for agriculture and stockbreeding. Specifically, this drug can be sprayed in order to, for example, deodorize a space. In the case of a site affected by a hoof disease and the like, absorbent cotton or gauze impregnated with this drug can be attached to the affected site. When this drug is used for hand sterilization, an aqueous solution thereof can be rubbed onto hands, for example. Medical devices and the like can be washed with this drug by spraying the liquid drug thereonto or immersing them into an aqueous solution of this drug. When used for machines used in the above-mentioned stables, such as an automobile, farm machines, and a forklift, the drug for agriculture and stockbreeding is, for example, atomized onto these machines, or used to wash these machines. When used as a measure to deodorize the above-mentioned animals, this drug can be, for example, atomized using an atomizer or the like or sprinkled using a sprinkler or the like. Eggs can be disinfected by, for example, applying this drug thereonto.

### Disinfectant for Agriculture and Stockbreeding

A disinfectant for agriculture and stockbreeding according to the present invention is characterized by including the drug for agriculture and stockbreeding according to the present invention. The drug for agriculture and stockbreeding according to the present invention can be used as, for example, a disinfectant. Conventionally, a variety of disinfectants have been used, but their disinfective effects are not satisfactory. Although the disinfective effects can be improved by increasing the concentrations thereof in some cases, safety problems may arise. A disinfectant that includes the drug for agriculture and stockbreeding according to the present invention exhibits sufficient disinfective effects even at a low concentration, and is thus highly safe.

### Disinfectant for Hand Sterilization for Agriculture and Stockbreeding

A disinfectant for hand sterilization for agriculture and stockbreeding according to the present invention is characterized by including the drug for agriculture and stockbreeding according to the present invention. The drug for agriculture and stockbreeding according to the present invention can be used as, for example, a disinfectant for hand sterilization for agriculture and stockbreeding to be used to sterilize hands, fingers and the like. A disinfectant for hand sterilization for agriculture and stockbreeding that includes the drug for agriculture and stockbreeding according to the present invention exhibits sufficient disinfective effects even at a low concentration, and is thus highly safe.

### Deodorant for Agriculture and Stockbreeding

A deodorant for agriculture and stockbreeding according to the present invention is characterized by including the drug for agriculture and stockbreeding according to the present invention. The drug for agriculture and stockbreeding according to the present invention can be used as, for example, a deodorant for agriculture and stockbreeding. In general, a quaternary ammonium salt is used as a common disinfectant component. In many cases, sufficient disinfective effects are not exhibited unless a quaternary ammonium salt is used at a high concentration, and a safety problem may arise. Also, a quaternary ammonium salt has no deodorizing effects, and thus a deodorizing component is mixed separately. Cyclodextrin is generally used as the deodorizing component. However, cyclodextrin has no ability to decompose a component responsible for unpleasant odors, and merely masks the component responsible for unpleasant odors, that is, cyclodextrin cannot remove unpleasant odors. For example, a deodorant for agriculture and stockbreeding that includes the drug for agriculture and stockbreeding according to the present invention has high disinfective effects, can decompose a substance responsible for unpleasant odors, and has high deodorizing effects.

### Disinfectant against Fungi for Agriculture and Stockbreeding

A disinfectant against fungi for agriculture and stockbreeding according to the present invention is characterized by including the drug for agriculture and stockbreeding according to the present invention. The disinfectant against fungi for agriculture and stockbreeding according to the present invention can be used as, for example, a disinfectant for sterilizing a site affected by fungi such as Trichophyton.

### Water Purification Agent for Agriculture and Stockbreeding

A water purification agent for agriculture and stockbreeding according to the present invention is characterized by including the drug for agriculture and stockbreeding according to the present invention. The water purification agent for agriculture and stockbreeding according to the present invention can kill, for example, bacteria such as Legionella growing in water to be used in the agriculture and stockbreeding. In addition, the drug for agriculture and stockbreeding according to the present invention does not corrode metals and generates no gases. Accordingly, a water purification agent for agriculture and stockbreeding that includes the drug for agriculture and stockbreeding according to the present invention can be used safely. The water purification agent for agriculture and stockbreeding according to the present invention can be used to, for example, kill bacteria contained in water or improve water quality. Accordingly, the water purification agent for agriculture and stockbreeding according to the present invention can also be referred to as, for example, a water disinfectant for agriculture and stock breeding, or a water quality conditioner for agriculture and stockbreeding.

### Usage of Drug for Agriculture and Stockbreeding

A usage of the drug for agriculture and stockbreeding according to the present invention is characterized by including a step of bringing a target and the drug for agriculture and stockbreeding according to the present invention into contact. With the usage of the drug for agriculture and stockbreeding according to the present invention, the target can be, for example, disinfected or deodorized.

### Examples

Hereinafter, examples of the present invention will be described. However, the present invention is not limited to the examples below.

### Example 1

1250 mg of sodium chlorite (NaClO₂, serving as a radical generation source), 1000 mg of benzethonium chloride (serving as a radical generating catalyst), and 7750 mg of disodium hydrogenphosphate (Na₂HPO₄, serving as a buffer and a liquid property-controlling agent) were placed in a mortar and mixed well. 1 g of the obtained mixture was charged into a tablet machine, and a solid drug according to the present invention was manufactured. Nine tablets were manufactured, and the weight of each tablet was measured and confirmed.

### Example 2

A solid drug (tablet) according to the present invention was manufactured in the same manner as in Example 1, except that no radical generating catalyst was used (that is, the addition amount of benzethonium chloride was set to zero).

### Example 3

A solid drug (tablet) according to the present invention was manufactured in the same manner as in Example 1, except that 100 mg of sodium carbonate (Na₂CO₃, serving as a stabilizer) was added in addition to 1250 mg of sodium chlorite (NaClO₂, serving as a radical generation source), 1000 mg of benzethonium chloride (serving as a radical generating catalyst), and 7750 mg of disodium hydrogenphosphate (Na₂HPO₄, serving as a buffer and a liquid property-controlling agent).

### Stability Test

Immediately after being manufactured, one tablet of each of the solid drugs (tablets) of Examples 1 to 3 was dissolved in 1000 mL of water to prepare a 100-ppm aqueous solution. The absorbance of this aqueous solution was measured, and was taken as a control (reference). On the other hand, the remaining drugs of Examples 1 to 3 were placed in an incubator (As One Corporation; trade name: EI-450V), and were incubated in an environment at a temperature of 40°C and a humidity of 75%. Each tablet was placed in a plastic bag, and then the plastic bag was sealed using a sealer. After incubating for predetermined number of days, the tablet was dissolved in 1000 mL of water to prepare an aqueous solution. Then, it was confirmed through an absorbance measurement and ion chromatography whether or not sodium chlorite (radical generation source) was degraded and to what extent sodium chlorite was degraded. In these embodiments, UV-2400PC (trade name) manufactured by Shimadzu Corporation was used for the absorbance measurement, and ICS-900 (trade name) manufactured by Thermo Scientific Dionex was used for the ion chromatography.

In all the cases of the tablets of Examples 1 to 3, it was confirmed that the concentration of sodium chlorite (radical generation source) did not decrease even 60 days after the start of the incubation, that is, sodium chlorite had not been degraded.

In the case of Example 2 in which benzethonium chloride (radical generating catalyst) was not used, sodium chlorite (radical generation source) was not degraded 120 days after the start of the incubation. On the other hand, in the case of Example 1 in which benzethonium chloride (radical generating catalyst) was used, it was confirmed through the ion chromatography that 40% of sodium chlorite (radical generation source) was degraded 120 days after the start of the incubation.

Furthermore, in the case of Example 3 in which sodium carbonate (stabilizer) was added, it was confirmed that sodium chlorite (radical generation source) was degraded more slowly than in Example 1, that is, the stability was improved.

### Disinfecting Ability Test

Each of the drugs (tablets) of Examples 1 to 3 was dissolved in water having a mass (weight) 1000 times as large as that of the tablet to manufacture a liquid drug (an aqueous solution of the tablet). This liquid drug was used in the same manner as described in Examples in Patent Literature 1 (Japanese Patent No. 6236057) and Patent Literature 2 (WO 2018/230743A1). As a result, it was confirmed that the drugs of Examples 1 and 3 had a disinfection function as in the cases of Patent Literature 1 and 2, and could be used as a disinfectant, a drug for agriculture and stockbreeding, and a drug for treating ulcerative colitis or suppressing symptoms thereof. On the other hand, the drug of Example 2 containing no radical generating catalyst (benzethonium chloride) had a disinfective function, but the disinfective function was weaker than those of Examples 1 and 3 due to slower radical generation speed.

### Example 4

Drugs were prepared in accordance with (1) and (2) below. A drug prepared in accordance with (1) below was a drug according to the present invention containing a radical generation source (sodium chlorite) and a radical generating catalyst (benzethonium chloride). A drug prepared in accordance with (2) below was a reference example containing a radical generation source (sodium chlorite) and no radical generating catalyst.
(1) 1.250 g of sodium chlorite (SIGMA-ALDORICH, product number: 244155-100G, content: 80 mass%) and 1.000 g of benzethonium chloride (FUJIFILM Wako Pure Chemical Corporation, product number: 025-11662) were mixed in a mortar, and were then placed in a glass beaker.
(2) 1.263 g of sodium chlorite (SIGMA-ALDORICH, 244155-100G, content: 80%) was placed in a glass beaker.

Each of the drugs of (1) and (2) above was placed in a desiccator manufactured by As One Corporation and heated to 40°C. Then, the desiccator was depressurized to 560 mmHG (560 Torr) using VACUUM PUMP manufactured by Bio Craft, and was then placed in an incubator at 40°C and left to stand for 110 days. As a result, the mass of the drug (1) changed from 2.250 g to 2.234 g. That is, the mass of the drug (1) that had been left to stand for 110 days was 99.30% of the original mass, and the mass hardly decreased. The mass of the drug (2) changed from 1.263 g to 1.262 g. That is, the mass of the drug (2) that had been left to stand for 110 days was 99.90% of the original mass, and there was substantially no difference therebetween.

As described above, it was confirmed from the results of this embodiment that both the masses of sodium chlorite and benzethonium chloride hardly decreased (that is, both were hardly degraded) under reduced pressure corresponding to about 75% of atmospheric pressure.

Although the present invention has been described with reference to the embodiments and examples, the present invention is not limited to the embodiments and examples described above. Various modifications that can be understood by a person skilled in the art can be made in the configurations and details of the present invention without departing from the scope of the present invention.

### Industrial Applicability

As described above, with the present invention, it is possible to provide a solid drug that is very convenient to transport and store, and a method for manufacturing the drug. Also, with the present invention, it is possible to provide, for example, a drug and a drug for agriculture and stockbreeding that are highly safe and highly disinfectant. The applications of the drug and the drug for agriculture and stockbreeding according to the present invention are not particularly limited. These drugs can be used for a wide variety of applications. The drug and the drug for agriculture and stockbreeding according to the present invention are significantly useful in, for example, the fields of agriculture, stockbreeding, and the like.

This application claims the benefit of priority from Japanese Patent Application No. 2019-181759 filed on October 1, 2019, the entire contents of which are incorporated herein by reference.

## Claims

1. A drug in a solid form comprising:
a radical generating catalyst; and
a radical generation source.

2. The drug according to claim 1,
wherein the radical generating catalyst includes an ammonium salt.

3. The drug according to claim 2,
wherein the ammonium salt is an ammonium salt represented by Chemical Formula (XI) below: 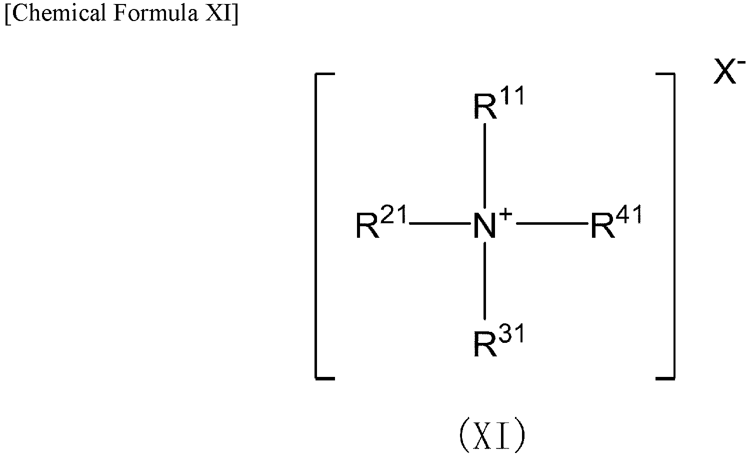 where
R¹¹, R²¹, R³¹, and R⁴¹ are independently a hydrogen atom or an aromatic ring, or an alkyl group that optionally includes an ether bond, a carbonyl group, an ester bond, or an amide bond, or an aromatic ring, and are optionally the same or different, or
two or more of R¹¹, R²¹, R³¹, and R⁴¹ are optionally linked to each other to form a ring structure together with N⁺ to which these groups link, the ring structure being optionally saturated or unsaturated, being optionally an aromatic ring or non-aromatic ring, and optionally having one or more substituents, and
X⁻ is an anion other than a peroxodisulfate ion.

4. The drug according to claim 3,
wherein the ammonium salt represented by Chemical Formula (XI) is an ammonium salt represented by Chemical Formula (XII) below: 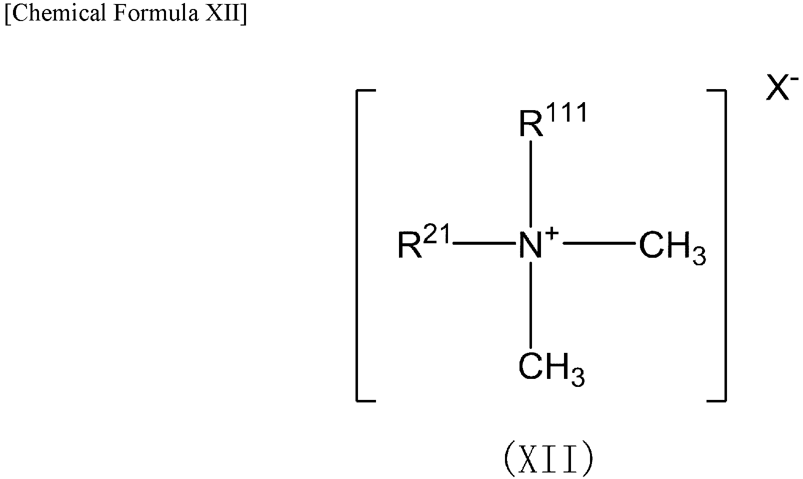 where
R¹¹¹ is an alkyl group having 5 to 40 carbon atoms and optionally includes an ether bond, a ketone (carbonyl group), an ester bond, or an amide bond, a substituent, or an aromatic ring, and
R²¹ and X⁻ are the same as those in Chemical Formula (XI) above.

5. The drug according to claim 4,
wherein the ammonium salt represented by Chemical Formula (XII) is an ammonium salt represented by Chemical Formula (XIII) below: 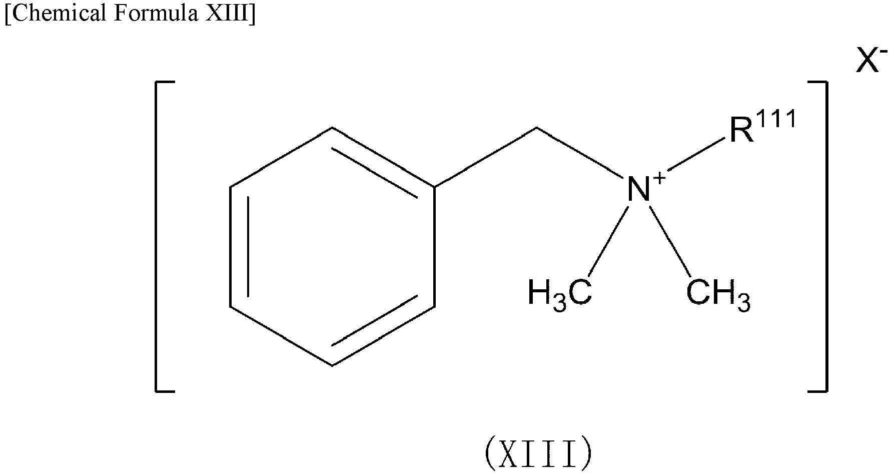 where
R¹¹¹ and X⁻ are the same as those in Chemical Formula (XII) above.

6. The drug according to claim 2,
wherein the ammonium salt is at least one selected from the group consisting of benzethonium chloride, benzalkonium chloride, hexadecyltrimethylammonium chloride, tetramethylammonium chloride, ammonium chloride, methylammonium chloride, tetrabutylammonium chloride, cetylpyridinium chloride, hexadecyltrimethylammonium bromide, dequalinium chloride, edrophonium, didecyldimethylammonium chloride, benzyltriethylammonium chloride, oxitropium, carbachol, glycopyrronium, safranine, sinapine, tetraethylammonium bromide, hexadecyltrimethylammonium bromide, suxamethonium, sphingomyelin, ganglioside GM1, denatonium, trigonelline, neostigmine, paraquat, pyridostigmine, phellodendrine, pralidoxime methyl iodide, betaine, betanin, bethanechol, betalain, lecithin, adenine, guanine, cytosine, thymine, uracil, and cholines.

7. The drug according to claim 4,
wherein the ammonium salt represented by Chemical Formula (XII) is benzethonium chloride.

8. The drug according to claim 3,
wherein the ammonium salt represented by Chemical Formula (XI) is an ammonium salt represented by Chemical Formula (XIV) below: 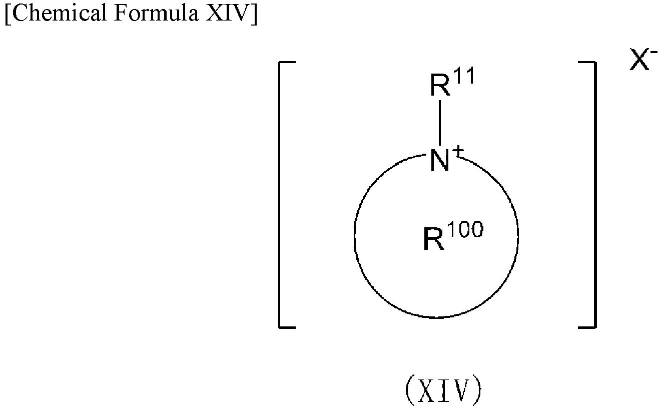 where
R¹⁰⁰ optionally forms a ring structure, the ring structure being optionally saturated or unsaturated, being optionally an aromatic ring or non-aromatic ring, and optionally having one or more substituents, and
R¹¹ and X⁻ are the same as those in Chemical Formula (XI) above.

9. The drug according to claim 3,
wherein the ammonium salt represented by Chemical Formula (XI) is an ammonium salt represented by Chemical Formula (XV) below: 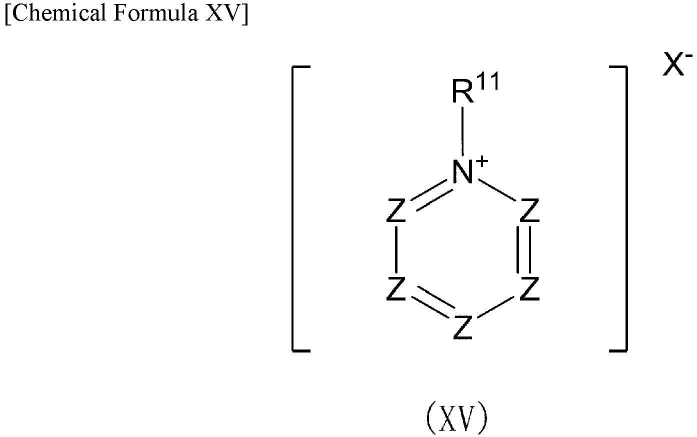 where
groups Z are independently CH or N, and are optionally the same or different, H in CH being optionally substituted with a substituent, and
R¹¹ and X⁻ are the same as those in Chemical Formula (XI) above.

10. The drug according to claim 3,
wherein the ammonium salt represented by Chemical Formula (XI) is an ammonium salt represented by Chemical Formula (XVI) below: 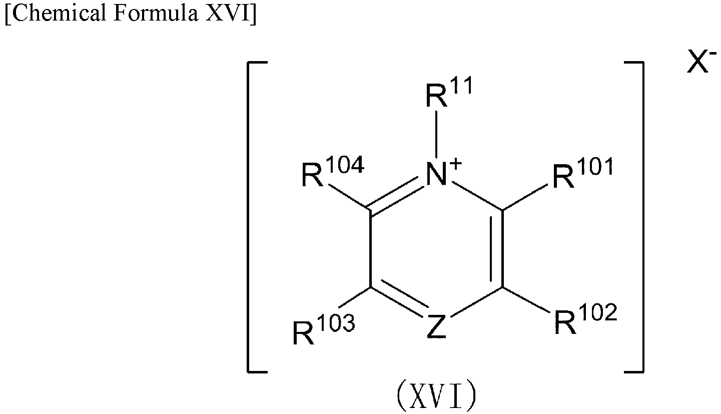 where
R¹⁰¹, R¹⁰², R¹⁰³, and R¹⁰⁴ are independently a hydrogen atom or a substituent, and are optionally the same or different, or
two or more of R¹⁰¹, R¹⁰², R¹⁰³, and R¹⁰⁴ are optionally linked to each other to form a ring structure together with N⁺ to which these groups link, the ring structure being optionally saturated or unsaturated, being optionally an aromatic ring or non-aromatic ring, and optionally having one or more substituents,
Z is CH or N, H in CH being optionally substituted with a substituent, and
R¹¹ and X⁻ are the same as those in Chemical Formula (XI) above.

11. The drug according to claim 3,
wherein the ammonium salt represented by Chemical Formula (XI) is an ammonium salt represented by Chemical Formula (XVII) below: 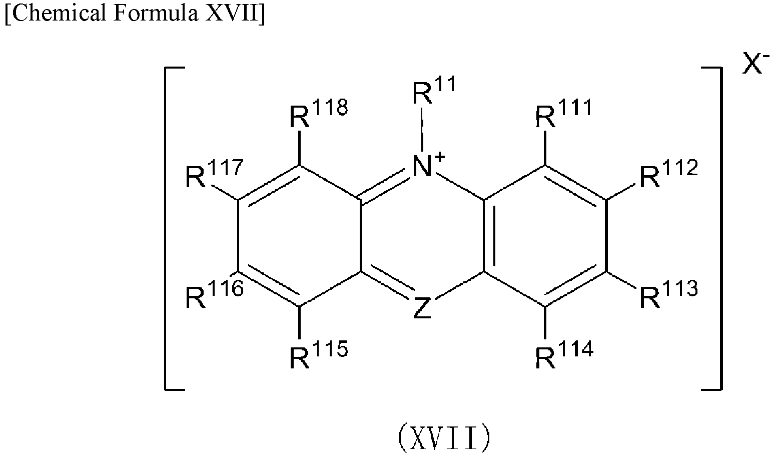 where
R¹¹¹ to R¹¹⁸ are independently a hydrogen atom or a substituent, and are optionally the same or different, or
two or more of R¹¹¹ to R¹¹⁸ are optionally linked to each other to form a ring structure, the ring structure being optionally an aromatic ring or non-aromatic ring, and optionally having one or more substituents,
Z is CH or N, H in CH being optionally substituted with a substituent, and
R¹¹ and X⁻ are the same as those in Chemical Formula (XI) above.

12. The drug according to claim 2,
wherein the ammonium salt is a NH₄⁺ salt.

13. The drug according to claim 12,
wherein the ammonium salt is NH₄Cl.

14. The drug according to any one of claims 2 to 12,
wherein the ammonium salt is a hexafluorophosphate salt of the ammonium.

15. The drug according to claim 1,
wherein the radical generating catalyst includes at least one selected from the group consisting of an amino acid, a protein, a peptide, a phospholipid, and salts thereof.

16. The drug according to claim 15,
wherein the amino acid is at least one selected from the group consisting of glycine, alanine, valine, leucine, isoleucine, serine, threonine, aspartic acid, glutamic acid, asparagine, glutamine, lysine, hydroxylysine, arginine, cysteine, cystine, methionine, phenylalanine, tyrosine, tryptophan, histidine, proline, and 4-hydroxyproline.

17. The drug according to claim 15 or 16,
wherein the peptide is at least one of oxidized glutathione (GSSG) and reduced glutathione (GSH).

18. The drug according to any one of claims 15 to 17,
wherein the phospholipid is at least one selected from the group consisting of phosphatidyl serine, phosphatidyl choline, phosphatidic acid, phosphatidylethanolamine, phosphatidyl glycerol, and cardiolipin.

19. The drug according to any one of claims 15 to 18,
wherein the radical generating catalyst further includes an ammonium salt.

20. The drug according to claim 19, wherein the ammonium salt is an ammonium salt according to any one of claims 3 to 14.

21. The drug according to any one of claims 1 to 18,
wherein the radical generating catalyst has a Lewis acidity of 0.4 eV or more.

22. The drug according to any one of claims 1 to 21,
wherein the radical generating catalyst has a Brønsted acid dissociation constant pKₐ of 5 or more.

23. The drug according to any one of claims 1 to 22,
wherein the radical generating catalyst catalyzes generation of a radical from the radical generation source in a non-acidic reaction system.

24. The drug according to any one of claims 1 to 22,
wherein the radical generating catalyst catalyzes generation of a radical from the radical generation source in an acidic reaction system.

25. The drug according to any one of claims 1 to 24,
wherein the radical generating catalyst catalyzes generation of a radical from the radical generation source in a solution.

26. The drug according to any one of claims 1 to 25,
wherein the radical generating catalyst catalyzes generation of a radical from the radical generation source in vivo.

27. The drug according to any one of claims 1 to 26,
wherein the radical generating catalyst catalyzes generation of a radical from the radical generation source in a digestive organ.

28. The drug according to claim 27,
wherein the digestive organ is at least one selected from the group consisting of an oral cavity, a pharynx, an esophagus, a stomach, a duodenum, a small intestine, and a large intestine.

29. The drug according to claim 27,
wherein the digestive organ is a large intestine.

30. The drug according to any one of claims 1 to 29,
wherein the radical generation source includes at least one selected from the group consisting of an oxoacid, an oxoacid salt, and an oxoacid ion.

31. The drug according to claim 30,
wherein the oxoacid is at least one selected from the group consisting of boric acid, carbonic acid, orthocarbonic acid, carboxylic acid, silicic acid, nitrous acid, nitric acid, phosphorous acid, phosphoric acid, arsenic acid, sulfurous acid, sulfuric acid, sulfonic acid, sulfinic acid, chromic acid, dichromic acid, permanganic acid, and a halogen oxoacid.

32. The drug according to claim 31,
wherein the halogen oxoacid is at least one selected from the group consisting of hypochlorous acid, chlorous acid, chloric acid, perchloric acid, hypobromous acid, bromous acid, bromic acid, perbromic acid, hypoiodous acid, iodous acid, iodic acid, and periodic acid.

33. The drug according to any one of claims 1 to 32,
wherein the radical generation source includes at least one selected from the group consisting of a halogen ion, a hypohalous acid ion, a halous acid ion, a halogen acid ion, and a perhalogen acid ion.

34. The drug according to claim 30,
wherein the oxoacid is a halogen oxoacid.

35. The drug according to claim 34,
wherein the halogen oxoacid is a chlorine oxoacid.

36. The drug according to any one of claims 1 to 29,
wherein the radical generation source is at least one selected from the group consisting of a halous acid, a halous acid ion, and a halous acid salt.

37. The drug according to claim 36,
wherein the halous acid is at least one selected from the group consisting of chlorous acid, bromous acid, and iodous acid.

38. The drug according to any one of claims 1 to 37,
wherein the radical generation source is a chlorous acid ion.

39. The drug according to any one of claims 1 to 38,
wherein a reaction rate constant (k_{cat}) of the radical generating catalyst for Chemical Reaction Formula (1b) below is 1.0×10⁻⁵ S⁻¹ or more: 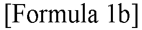 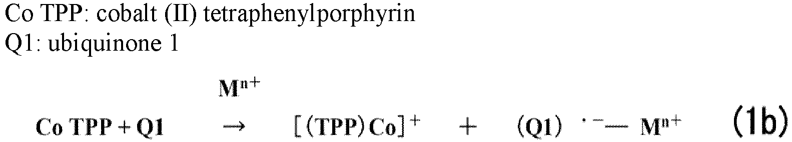 where
Mⁿ⁺ represents the radical generating catalyst,
CoTPP represents cobalt (II) tetraphenylporphyrin,
Q1 represents ubiquinone 1,
[(TPP)Co]⁺ represents a cobalt (III) tetraphenylporphyrin cation, and
(Q1)^{·-} represents a ubiquinone 1 anion radical.

40. The drug according to any one of claims 1 to 39, further comprising
at least one of a buffer and a liquid property-controlling agent.

41. The drug according to any one of claims 1 to 40,
wherein the radical generating catalyst and the radical generation source are separated by another layer.

42. The drug according to claim 41,
wherein the other layer is a coating layer or a microcapsule layer.

43. A drug in a solid form comprising:
at least one of a buffer and a liquid property-controlling agent; and
a radical generation source.

44. The drug according to claim 43,
wherein the radical generation source is the radical generation source according to any one of claims 30 to 38.

45. The drug according to any one of claims 1 to 44, further comprising
a stabilizer.

46. The drug according to claim 45,
wherein the stabilizer is a substance that suppresses or prevents generation of a radical from the radical generation source in the drug in a solid form.

47. The drug according to claim 45 or 46,
wherein the stabilizer is a substance capable of oxidizing or reducing a radical generated from the radical generation source.

48. The drug according to any one of claims 45 to 47,
wherein the stabilizer is at least one of a carbonate and a hydrogencarbonate.

49. The drug according to any one of claims 45 to 48,
wherein the stabilizer is sodium carbonate.

50. The drug according to any one of claims 1 to 49,
which is in a form of a tablet.

51. The drug according to any one of claims 1 to 50,
which is dissolved or dispersed in a liquid medium before use.

52. The drug according to claim 51,
wherein the liquid medium is at least one of water and an organic solvent.

53. The drug according to claim 51,
wherein the liquid medium is water.

54. The drug according to any one of claims 51 to 53,
wherein a solution obtained by dissolving or dispersing the drug in the liquid medium is non-acidic.

55. The drug according to any one of claims 1 to 54, which is a disinfectant.

56. The drug according to any one of claims 1 to 55, which is to be used in vivo.

57. The drug according to any one of claims 1 to 56, which is to be used in a digestive organ.

58. The drug according to claim 57,
wherein the digestive organ is at least one selected from the group consisting of an oral cavity, a pharynx, an esophagus, a stomach, a duodenum, a small intestine, and a large intestine.

59. The drug according to claim 58,
wherein the digestive organ is a large intestine.

60. The drug according to any one of claims 56 to 59, which is to be used to treat ulcerative colitis or suppress symptoms thereof.

61. The drug according to any one of claims 1 to 60, which is a drug for agriculture and stockbreeding.

62. The drug for agriculture and stockbreeding according to claim 61,
wherein the drug for agriculture and stockbreeding is at least one selected from the group consisting of a disinfectant for agriculture, an antiviral agent for agriculture, a deodorant for agriculture, a pesticide for agriculture, a repellent for agriculture, a soil conditioner for agriculture, a disinfectant for stockbreeding, an antiviral agent for stockbreeding, a deodorant for stockbreeding, a pesticide for stockbreeding, a repellent for stockbreeding, and a soil conditioner for stockbreeding.

63. A method for manufacturing the drug according to any one of claims 1 to 62, comprising
a pressing step of pressing a composition containing constituent components of the drug into a solid form.

64. A water purification method comprising
a step of adding the drug according to claims 1 to 62, or a drug-containing solution containing the drug, into water.

65. The water purification method according to claim 64, wherein the water purification includes at least one of disinfection and deodorization of the water.
